(19) Europäisches Patentamt | European Patent Office | Office européen des brevets

(11) **EP 3 894 600 B1**

(12) **EUROPEAN PATENT SPECIFICATION**

(45) Date of publication and mention
of the grant of the patent:
**04.01.2023 Bulletin 2023/01**

(21) Application number: **19824204.2**

(22) Date of filing: **11.12.2019**

(51) International Patent Classification (IPC):
***C12Q 1/6886*** *(2018.01)*

(52) Cooperative Patent Classification (CPC):
(C-Sets available)
**C12Q 1/6886; C12Q 1/6851;** C12Q 2600/156

(Cont.)

(86) International application number:
**PCT/IB2019/060638**

(87) International publication number:
**WO 2020/121206 (18.06.2020 Gazette 2020/25)**

(54) **METHOD FOR QUANTIFYING GENE FUSION DNA**

VERFAHREN ZUR QUANTIFIZIERUNG VON GENFUSIONS-DNA

PROCÉDÉ DE QUANTIFICATION LA FUSION DE GÈNES D'ADN

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.12.2018 US 201862778537 P**
**17.12.2018 US 201862780807 P**

(43) Date of publication of application:
**20.10.2021 Bulletin 2021/42**

(73) Proprietor: **INIVATA LTD.**
**Cambridge CB22 3FH (GB)**

(72) Inventors:
• **HOWARTH, Karen**
**Cambridge CB22 3FH (GB)**
• **WOODHOUSE, Samuel**
**Cambridge CB22 3FH (GB)**
• **FORSHEW, Tim**
**Cambridge CB22 3FH (GB)**
• **PLAGNOL, Vincent**
**Cambridge CB22 3FH (GB)**

(74) Representative: **Leonard, Thomas Charles et al**
**Kilburn & Strode LLP**
**Lacon London**
**84 Theobalds Road**
**London WC1X 8NL (GB)**

(56) References cited:
**WO-A1-2008/144841**     **WO-A1-2018/005983**
**WO-A1-2018/229514**

• **METZLER M ET AL: "Asymmetric
multiplex-polymerase chain reaction - a high
throughput method for detection and sequencing
genomic fusion sites in t(4;11)", BRITISH
JOURNAL OF HAEMATOLOGY,
WILEY-BLACKWELL PUBLISHING LTD, GB, vol.
124, 23 September 2004 (2004-09-23), pages
47-54, XP008122029, ISSN: 0007-1048**

Remarks:
The complete document including Reference
Table(s) and the Sequence Listing(s) can be
downloaded from the EPO website

(52) Cooperative Patent Classification (CPC): (Cont.)

C-Sets
**C12Q 1/6851, C12Q 2537/143, C12Q 2545/101**

**Description**

## BACKGROUND

Gene fusions involving kinase genes (e.g., RET, ROS1 BRAF, NTRK1, NTRK3, ALK, and RAF1, among many others) are thought to drive growth and survival of a subset of cancers (see, e.g., Gao et al, Cell Reports 2018 23: 227-238). Targeted inhibition of aberrant signalling caused by such fusions can produce a dramatic and durable response in patients that have those cancers.

In theory, the amount of fusion DNA in the cell-free fraction of a patient's bloodstream (i.e., cfDNA) should correlate with disease severity for those cancers that are associated with the fusion, e.g., a subset of non-small cell lung cancers. Thus, tracking the amount of fusion DNA over time could be used to, for example, determine if a treatment is working. Assays for accurately quantifying the amount of a particular fusion sequence in a sample are well known. For example, qPCR or Invader assay could be used. However, in the clinic, such assays are not straightforward to implement because different patients have different fusions and, even if the genes that are fused together in a patient's cancer are known, the genes can be fused in different places. Such analyses are complicated by the fact that cfDNA is highly fragmented and, as such, samples that contain cfDNA are not amenable to analysis by some of the methods that are used to analyse samples that contain an intact genome. Thus, identifying and quantifying gene fusions in cfDNA would logically be implemented in two steps, where the first step involves sequencing a patient's cfDNA to identify which genes are fused as well as the sequence at the junction of the fusion, and a second step that involves quantifying the amount of fusion DNA in the cfDNA (see, e.g., Harris et al, Nature Scientific Reports 2016 6: 29831). The problem with this approach is that the latter step is patient-specific in the sense that most reliable quantification methods (e.g., qPCR or Invader, for example) only work if primers that flank the fusion junction are used. Thus, in order to implement the conventional workflow, one would have to carefully select a custom primer pair for each patient being tested, before quantifying the amount of fusion DNA. This is problematic because performing patient-specific assays using, e.g., custom sets of primers, is time consuming, inefficient and creates a significant potential for human error. Therefore, such assays should be avoided in the clinic, where robust, high-throughput methods are required.

Therefore, there is still a need for methods for quantifying the amount of fusion DNA that do not require patient-specific customization. One solution is the amplification with a set of tiled primers spanning the fusion region. WO2018005983 describes a method for detecting a gene fusion by amplifying nucleic acid with a series of tiled forward target- specific primers and a reverse universal primer. Fusion percentages were calculated based on total fusion reads detected for individual primers. WO2008144841 discloses a method of identifying a gene breakpoint with one or more forward primers directed to a DNA region located 5' relative to the gene breakpoint and one or more reverse primers directed to a DNA region located 3' relative to the gene breakpoint. WO2018229514 discloses detection of genomic MLL-AF4 fusion sites by asymmetric multiplex PCR with primers that cover the complete breakpoint cluster region of both genes. There is however need of a method that at the same time provides a more reliable quantification.

## SUMMARY

Provided herein is a way to quantify DNA fusion molecules in a sample. In some embodiments, the method may comprise: (a) combining a test sample comprising cell-free DNA (cfDNA) obtained from the bloodstream of a human subject with a set of primers and a polymerase to produce a reaction mix, wherein the set of primers comprises: i. at least 20 fusion-specific forward primers, wherein the fusion-specific forward primers tile across the same strand of a first region in a reference human genome, ii at least 20 fusion-specific reverse primers, wherein the fusion-specific reverse primers tile across the same strand in a second region of the reference human genome, and wherein the first and second regions are on different chromosomes or are on the same chromosome but spaced apart by at least 10 kb; and iii. a reference primer pair, wherein the reference primer pair amplifies a reference region of the genome, wherein the region amplified by the reference primer pair is in the range of 40 bp to 160 bp; (b) thermocycling the reaction mix to produce PCR products that comprise: i. a reference amplicon that is produced by the reference primer pair, and ii. one or more fusion amplicons that are produced using the fusion-specific primers from fusion molecules in the cfDNA, wherein the fusion molecules correspond to a genomic rearrangement that fuses the first region with the second region in at least some cells of the subject; and (c) sequencing the PCR products of (b) or amplification products thereof to produce sequence reads; and (d) quantifying the relative abundance of the fusion molecules in the test sample by comparing the number of sequence reads corresponding to fusion molecules with of the number of sequence reads corresponding to the reference region, to produce a ratio.

The method solves a problem because it provides a way to identify and quantify gene fusions in the same assay. Importantly, once identified, a specific gene fusion that has been previously identified can by quantified in the future using the same reagents (i.e., the same set of primers), without any patient-specific customization. As such, the same method can be performed on different samples collected from the same patient at different time-points, and the abundance of the fusion molecules in the sample collected at the first time-point can be compared to the abundance of the fusion molecules in the sample collected at the second time-point in order to determine if the amount of fusion molecules has changed. In these embodiments, the method may comprise separately analysing a first test sample and a second test sample using the present method to obtain a first ratio indicating the abundance of the fusion molecules in the first sample and a second ratio indicating the abundance of the fusion molecules in the second test sample, where the first and second test samples are obtained from the same subject at different time points. The first and second ratios can be compared to determine if the abundance of the fusion molecules has changed over time.

Illustrated by example, tens, hundreds or thousands of different samples of cfDNA from different patients can be analysed using the present method. Patients that have a gene fusion can be identified, the genes that have been fused in those patients can be determined, the fusion junction can be identified and the quantity of fusion DNA in the patient's cfDNA can be calculated. In theory, the same assay can be applied to every sample, without customizing the PCR for a particular patient. Moreover, the quantity of fusion DNA in the portion of patients that have a fusion can be re-tested at a later date using exactly the same assay.

## BRIEF DESCRIPTION OF THE FIGURES

The skilled artisan will understand that the drawings, described below, are for illustration purposes only. The drawings are not intended to limit the scope of the present teachings in any way.

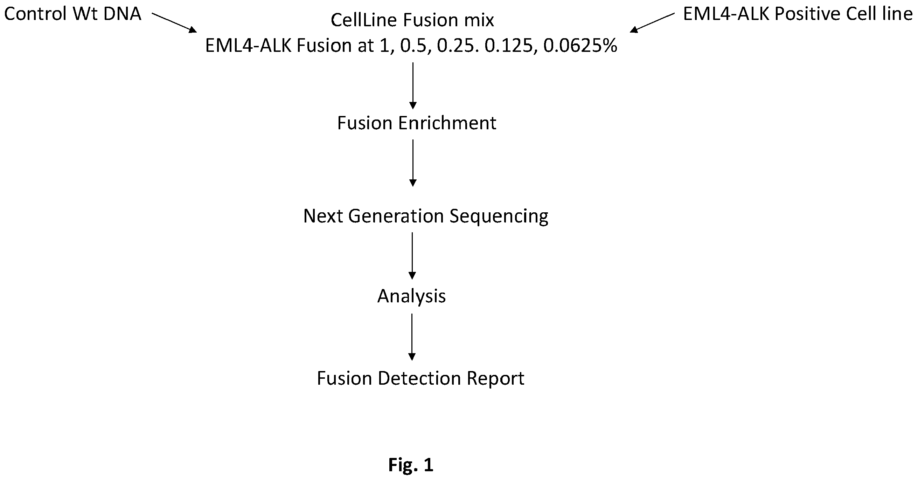

**FIG. 1:** Cell line fusion mix (custom product Horizon Discovery Group) consisting of a mixture of EML4-ALK fusion-positive DNA and normal (fusion-negative) DNA was serially diluted to achieve allelic fractions of 1%, 0.5%, 0.25%, 0.125% and 0.0625%. Fusion-negative human placental DNA (bioline) was added to maintain the genome input copy number constant at 4000 input copies. Fusion enrichment is achieved by selective PCR and a second PCR ensures the addition of barcoded illumina adapters. Fusion genes are decoded by next-generation sequencing e.g. on the NextSeq 500 Illumina platform. Sequencing data is screened for the presence of fusion genes using the described bioinformatic pipeline and data is published in a fusion detection report.

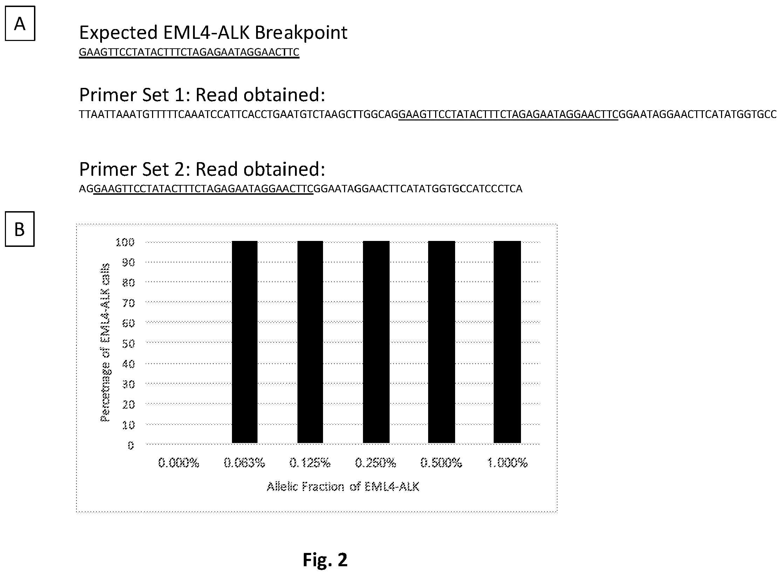

**FIG. 2:** The sequence of the EML4-ALK gene fusion in the fusion-positive material from Horizon is known (expected EML4-ALK breakpoint). **A.** Different combinations of adjacent primers are able to amplify the gene fusion. Two types of reads, containing the fusion breakpoint, are obtained as a result of amplification of the fusion by different primer pairs. Both reads contain the expected fusion gene sequence as well as flanking DNA sequences of different lengths. From top to bottom: SEQ ID NOS: 1-3. **B.** Fusion detection was performed on three replicates at each of the allelic frequencies. Fusions were detected at all allelic fractions in all replicates.

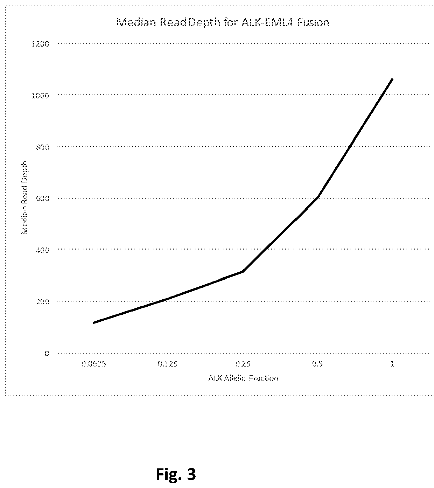

**FIG. 3:** Median read depth obtained for an EML4-ALK fusion at 0.0625, 0.125, 0.25, 0.5 and 1% allelic fraction. The median of the number of fusion reads detected in the three replicates was calculated and plotted against the different allelic fractions.

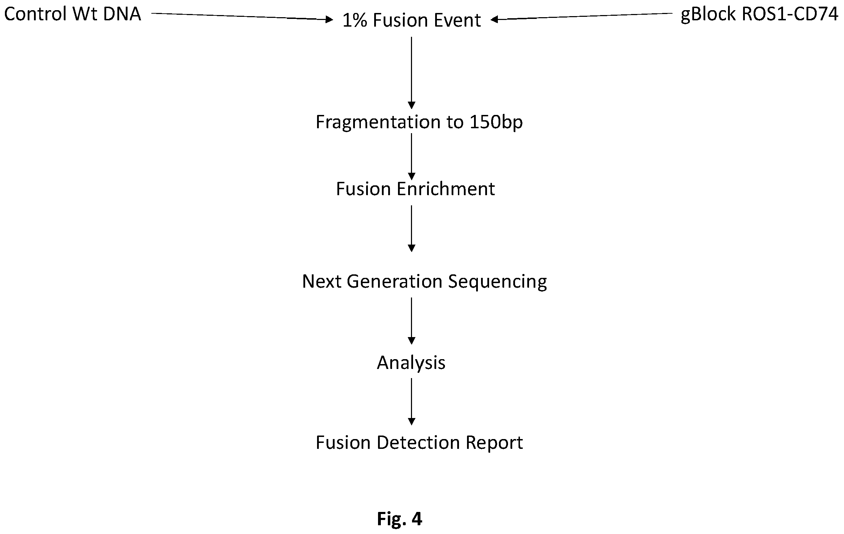

**FIG. 4:** Experiment determining ROS1 Fusion. To test the detection of fusion genes between ROS1 and CD74, a 500 bp fragment of synthetic DNA (gblock) that contains the sequence of a published CD74-ROS1 gene fusion was synthesized by IDT. The synthetic gblock was fragmented by sonication (Covaris) to an average of 150bp and added to sheared fusion-negative human placental DNA to achieve an allelic fraction of 1% at 4000 input copies. The fusion gene was amplified by selective PCR and decoded on the NextSeq500 (Illumina). Sequencing data is screened for the presence of fusion genes using the described bioinformatic pipeline and data is published in a fusion detection report.

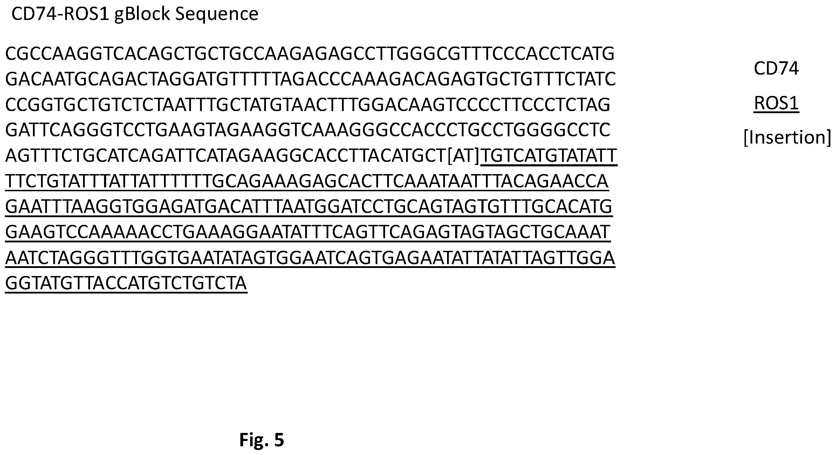

**FIG. 5:** The sequence of the synthesised CD74-ROS1 fusion containing gblock is depicted. The gblock was fragmented to an average of 150 bp prior to inputting into the assay. SEQ ID NO: 5.

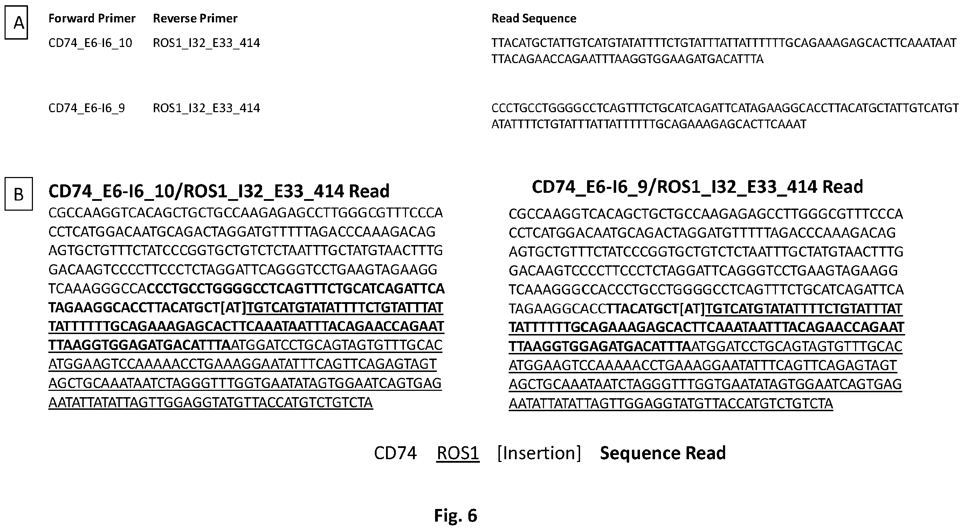

**FIG. 6:** Next Generation sequencing reads obtained from CD74-ROS1 gBlock. **A.** Two combinations of forward and reverse primers amplified the fusion gene. SEQ ID NOS: 5 and 6. **B.** The sequence of the read detected for each is shown. The sequence of the read is shown in bold letters within the geneblock sequence. SEQ ID NOS: 7 and 8.

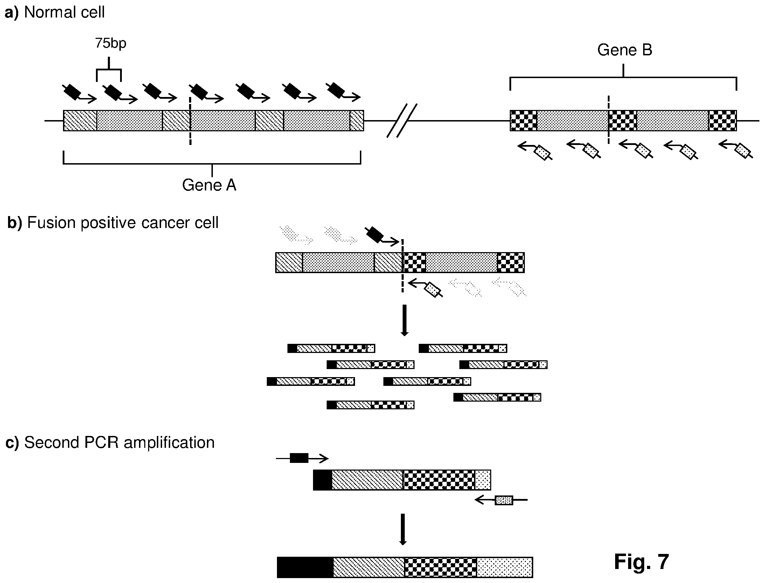

**FIG. 7:** Example 2-step workflow showing multiplex PCR conducted with primers that tile genes of interest at intervals (75bp in this example). Gene A is tiled only with forward primers and Gene B is tiled only with reverse primers. The primers contain a universal primer site (UPS) (for example part of an Illumina adaptor sequence) at the 5' end and a gene specific sequence at the 3' end. A. in normal cells that do not have a gene fusion, PCR amplification does not occur as the distance between the genes is too great. **B.** in fusion-positive cancer cells, Genes A and B are brought into close proximity with one another (for example within 150bp) so a product is generated by PCR amplification **C**. The presence of the UPSs (such as UPSs incorporated in partial sequencing adaptors, such as partial Illumina adaptors) allows the construction of complete sequencing adaptors (such as Illumina adaptors) in a second round of PCR. This second round of PCR uses primers that anneal to the UPS element of the original primers (at the 3' end of the primer) and contain the rest of the sequencing adaptor (at the 5' end of the primer).

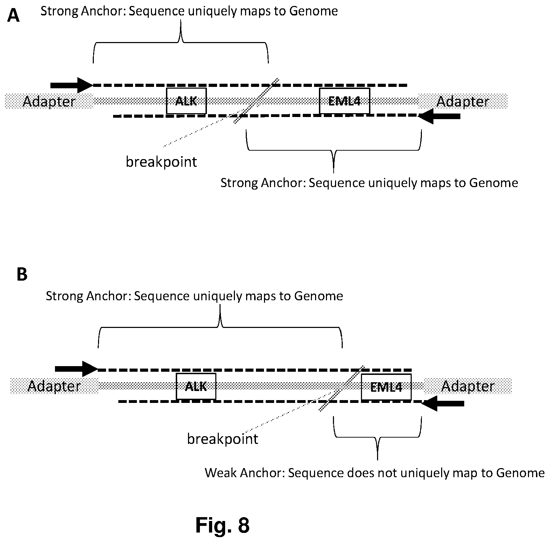

**FIG. 8:** Bioinformatic method for calling gene fusions: Amplicons are generated by two primer pairs amplifying a fusion event which are then sequenced (dotted line indicates read) by NGS (Black Arrows indicate sequencing primers). The analysis method involves determining the minimum number of base pairs that need to be sequenced (for each primer site) to uniquely match a target region. A strong anchor has sufficient base pairs sequenced to uniquely match a target region, a weak anchor does match a target region but also matches other regions in the reference genome, it therefore does not uniquely match the target region. The method uses the known primer binding locations to determine the expected sequence within the reads which removes the need for aligning reads to the entire reference genome. **A.** An amplicon has two strong anchors with both the ALK and EML4 portions of read uniquely matching an ALK or EML4 reference sequence. **B.** An amplicon has one strong anchor and one weak anchor. The ALK portion of the read uniquely matches a target region, EML4 does not uniquely match the reference genome.

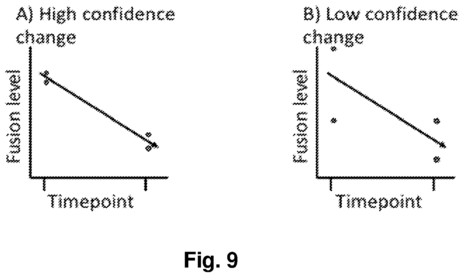

**FIG. 9** shows two graphs illustrating how replicates can be used to assign a confidence to a determination that the amount of a fusion DNA has changed over time.

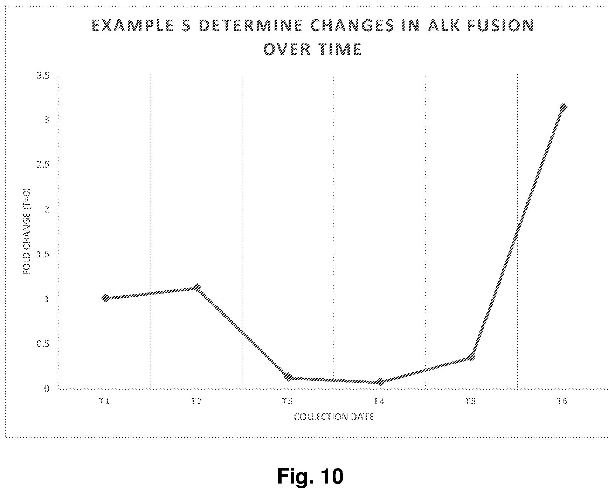

**FIG. 10** is a graph showing that the amount of ALK fusion molecules in cfDNA changes over time.

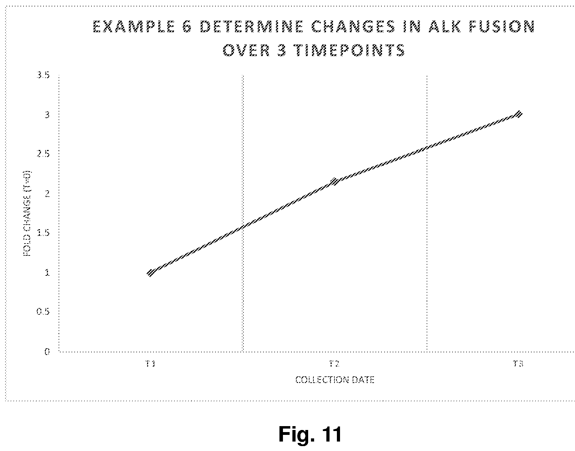

**FIG. 11** is a graph showing that the amount of an ALK fusion molecules in cfDNA can change over three time points.

## DEFINITIONS

Unless otherwise defined, all technical and scientific terms used herein have the same meaning as commonly understood by one of ordinary skill in the art to which this invention belongs. Still, certain elements are defined for the sake of clarity and ease of reference.

Terms and symbols of nucleic acid chemistry, biochemistry, genetics, and molecular biology used herein follow those of standard treatises and texts in the field, e.g. Kornberg and Baker, DNA Replication, Second Edition (W.H. Freeman, New York, 1992); Lehninger, Biochemistry, Second Edition (Worth Publishers, New York, 1975); Strachan and Read, Human Molecular Genetics, Second Edition (Wiley-Liss, New York, 1999); Eckstein, editor, Oligonucleotides and Analogs: A Practical Approach (Oxford University Press, New York, 1991); Gait, editor, Oligonucleotide Synthesis: A Practical Approach (IRL Press, Oxford, 1984); and the like.

The term "nucleic acid sample," as used herein, denotes a sample containing nucleic acids. Nucleic acid samples used herein may be complex in that they contain multiple different molecules that contain sequences. Genomic DNA samples from a mammal (e.g., mouse or human) are types of complex samples. Complex samples may have more than about $10^4$, $10^5$, $10^6$ or $10^7$, $10^8$, $10^9$ or $10^{10}$ different nucleic acid molecules. Any sample containing nucleic acid, e.g., genomic DNA from tissue culture cells or a sample of tissue, may be employed herein.

The term "oligonucleotide" as used herein denotes a multimer of nucleotides of about 2 to 200 nucleotides, up to 500 nucleotides in length. Oligonucleotides may be synthetic or may be made enzymatically, and, in some embodiments, are 30 to 150 nucleotides in length. Oligonucleotides may contain ribonucleotide monomers (i.e., may be oligoribonucleotides) or deoxyribonucleotide monomers, or both ribonucleotide monomers and deoxyribonucleotide monomers. An oligonucleotide may be 10 to 20, 21 to 30, 31 to 40, 41 to 50, 51 to 60, 61 to 70, 71 to 80, 80 to 100, 100 to 150 or 150 to 200 nucleotides in length, for example.

"Primer" means an oligonucleotide, either natural or synthetic, that is capable, upon forming a duplex with a polynucleotide template, of acting as a point of initiation of nucleic acid synthesis and being extended from its 3' end along the template so that an extended duplex is formed. The sequence of nucleotides added during the extension process is determined by the sequence of the template polynucleotide. Primers are extended by a DNA polymerase. Primers are generally of a length compatible with their use in synthesis of primer extension products, and are usually in the range of 8 to 200 nucleotides in length, such as 10 to 100 or 15 to 80 nucleotides in length. A primer may contain a 5' tail that does not hybridize to the template. Primers are usually single-stranded for maximum efficiency in amplification, but may alternatively be double-stranded or partially double-stranded. Thus, a "primer" is complementary to a template, and complexes by hydrogen bonding or hybridization with the template to give a primer/template complex for initiation of synthesis by a polymerase, which is extended by the addition of covalently bonded bases linked at its 3' end complementary to the template in the process of DNA synthesis.

In certain cases, a set of primers may be designated as being "forward" or "reverse" primers. The assignment of a primer as a forward or reverse primer is arbitrary and does not imply any particular orientation, function or structure relative to a coding sequence or strand. Rather, the terms simply denote that there are two separate groups of primers where, within each group, all the primers hybridize to the same strand in a defined region. The terms "forward" and "reverse" could be changed to "first" and "second" (or, in some embodiments, "kinase-specific" and "fusion partner-specific") in any embodiment. As will be described in greater below, the sets of forward and reverse primers used in the present method do not produce a product in a PCR reaction unless there is a rearrangement in the genome. Forward and reverse primers may hybridize to different chromosomes or different chromosome arms. For some fusions, a forward primer may hybridize to the bottom strand and a reverse primer may hybridize to the top strand, whereas in other fusions, a forward primer may hybridize to the top strand and a reverse primer may hybridize to the bottom strand.

The term "hybridization" or "hybridizes" refers to a process in which a region of nucleic acid strand anneals to and forms a stable duplex, either a homoduplex or a heteroduplex, under normal hybridization conditions with a second complementary nucleic acid strand, and does not form a stable duplex with unrelated nucleic acid molecules under the same normal hybridization conditions. The formation of a duplex is accomplished by annealing two complementary nucleic acid strand region in a hybridization reaction. The hybridization reaction can be made to be highly specific by adjustment of the hybridization conditions under which the hybridization reaction takes place, such that two nucleic acid strands will not form a stable duplex, e.g., a duplex that retains a region of doublestrandedness under normal stringency conditions, unless the two nucleic acid strands contain a certain number of nucleotides in specific sequences which are substantially or completely complementary. "Normal hybridization or normal stringency conditions" are readily determined for any given hybridization reaction. See, for example, Ausubel et al., Current Protocols in Molecular Biology, John Wiley & Sons, Inc., New York, or Sambrook et al., Molecular Cloning: A Laboratory Manual, Cold Spring Harbor Laboratory Press. As used herein, the term "hybridizing" or "hybridization" refers to any process by which a strand of nucleic acid binds with a complementary strand through base pairing.

A nucleic acid is considered to be "selectively hybridizable" to a reference nucleic acid sequence if the two sequences specifically hybridize to one another under moderate to high stringency hybridization conditions. Moderate and high stringency hybridization conditions are known (see, e.g., Ausubel, et al., Short Protocols in Molecular Biology, 3rd ed., Wiley & Sons 1995 and Sambrook et al., Molecular Cloning: A Laboratory Manual, Third Edition, 2001 Cold Spring Harbor, N.Y.).

The term "duplex," or "duplexed," as used herein, describes two complementary polynucleotide region that are base-paired, i.e., hybridized together.

"Genetic locus," "locus,", "locus of interest", "region" or "segment" in reference to a genome or target polynucleotide, means a contiguous sub-region or segment of the genome or target polynucleotide. As used herein, genetic locus, locus, or locus of interest may refer to the position of a nucleotide, a gene or a portion of a gene in a genome or it may refer to any contiguous portion of genomic sequence whether or not it is within, or associated with, a gene, e.g., a coding sequence. A genetic locus, locus, or locus of interest can be from a single nucleotide to a segment of a few hundred or a few thousand nucleotides in length or more. In general, a locus of interest will have a reference sequence associated with it (see description of "reference sequence" below).

The terms "reference sequence" and "reference region", as used herein, refer to a known nucleotide sequence, e.g. a chromosomal region or genome whose sequence is deposited at NCBI's Genbank database or other databases, for example. A reference sequence can be a wild type sequence.

The terms "plurality", "population" and "collection" are used interchangeably to refer to something that contains at least 2 members. In certain cases, a plurality, population or collection may have at least 10, at least 100, at least 1,000, at least 10,000, at least 100,000, at least $10^6$, at least $10^7$, at least $10^8$ or at least $10^9$ or more members.

The term "variable", in the context of two or more nucleic acid sequences that are variable, refers to two or more nucleic acids that have different sequences of nucleotides relative to one another. In other words, if the polynucleotides of a population have a variable sequence, then the nucleotide sequence of the polynucleotide molecules of the population may vary from molecule to molecule. The term "variable" is not to be read to require that every molecule in a population has a different sequence to the other molecules in a population.

The term "sequence variation", as used herein, is a variant that is present a frequency of less than 50%, relative to other molecules in the sample, where the other molecules in the sample are substantially identical to the molecules that contain the sequence variation. In some cases, a particular sequence variation may be present in a sample at a frequency of less than 20%, less than 10%, less than 5%, less than 1% or less than 0.5%. A sequence variation may be generated somatic mutation. However, in other embodiments, sequence variation may be derived from a developing fetus, a SNP or an organ transplant, for example.

The term "nucleic acid template" is intended to refer to the initial nucleic acid molecule that is copied during amplification. Copying in this context can include the formation of the complement of a particular single-stranded nucleic acid. The "initial" nucleic acid can comprise nucleic acids that have already been processed, e.g., amplified, extended, labeled with adaptors, etc.

The term "tailed", in the context of a tailed primer or a primer that has a 5' tail, refers to a primer that has a region (e.g., a region of at least 12-50 nucleotides) at its 5' end that does not hybridize or partially hybridizes to the same target as the 3' end of the primer.

The term "initial template" refers to a sample that contains a target sequence to be amplified. The term "amplifying" as used herein refers to generating one or more copies of a target nucleic acid, using the target nucleic acid as a template.

A "polymerase chain reaction" or "PCR" is an enzymatic reaction in which a specific template DNA is amplified using one or more pairs of sequence specific primers.

"PCR conditions" are the conditions in which PCR is performed, and include the presence of reagents (e.g., nucleotides, buffer, polymerase, etc.) as well as temperature cycling (e.g., through cycles of temperatures suitable for denaturation, renaturation and extension), as is known in the art.

The term "next generation sequencing" refers to the so-called highly parallelized methods of performing nucleic acid sequencing and comprises the sequencing-by-synthesis or sequencing-by-ligation platforms currently employed by Illumina, Life Technologies, Pacific Biosciences and Roche, etc. Next generation sequencing methods may also include, but not be limited to, nanopore sequencing methods such as offered by Oxford Nanopore or electronic detection-based methods such as the Ion Torrent technology commercialized by Life Technologies.

The term "sequence read" refers to the output of a sequencer. A sequence read typically contains a string of Gs, As, Ts and Cs, of 50-1000 or more bases in length and, in many cases, each base of a sequence read may be associated with a score indicating the quality of the base call.

The terms "assessing the presence of" and "evaluating the presence of" include any form of measurement, including determining if an element is present and estimating the amount of the element. The terms "determining", "measuring", "evaluating", "assessing" and "assaying" are used interchangeably and include quantitative and qualitative determinations. Assessing may be relative or absolute. "Assessing the presence of" includes determining the amount of something present, and/or determining whether it is present or absent.

If two nucleic acids are "complementary," they hybridize with one another under high stringency conditions. The term "perfectly complementary" is used to describe a duplex in which each base of one of the nucleic acids base pairs with a complementary nucleotide in the other nucleic acid. In many cases, two sequences that are complementary have at least 10, e.g., at least 12 or 15 nucleotides of complementarity.

An "oligonucleotide binding site" refers to a site to which an oligonucleotide hybridizes in a target polynucleotide. If an oligonucleotide "provides" a binding site for a primer, then the primer may hybridize to that oligonucleotide or its complement.

The term "strand" as used herein refers to a nucleic acid made up of nucleotides covalently linked together by covalent bonds, e.g., phosphodiester bonds. In a cell, DNA usually exists in a double-stranded form, and as such, has two complementary strands of nucleic acid referred to herein as the "top" and "bottom" strands. In certain cases, complementary strands of a chromosomal region may be referred to as "plus" and "minus" strands, the "first" and "second" strands, the "coding" and "noncoding" strands, the "Watson" and "Crick" strands or the "sense" and "antisense" strands. The assignment of a strand as being a top or bottom strand is arbitrary and does not imply any particular orientation, function or structure. The nucleotide sequences of the first strand of several exemplary mammalian chromosomal regions (e.g., BACs, assemblies, chromosomes, etc.) is known, and may be found in NCBI's Genbank database, for example.

The term "extending", as used herein, refers to the extension of a primer by the addition of nucleotides using a polymerase. If a primer that is annealed to a nucleic acid is extended, the nucleic acid acts as a template for extension reaction.

The term "sequencing," as used herein, refers to a method by which the identity of at least 10 consecutive nucleotides (e.g., the identity of at least 20, at least 50, at least 100 or at least 200 or more consecutive nucleotides) of a polynucleotide is obtained.

As used herein, the terms "cell-free DNA from the bloodstream" "circulating cell-free DNA" and "cell-free DNA" ("cfDNA") refers to DNA that is circulating in the peripheral blood of a patient. The DNA molecules in cell-free DNA may have a median size that is below 1 kb (e.g., in the range of 50 bp to 500 bp, 80 bp to 400 bp, or 100-1,000bp), although fragments having a median size outside of this range may be present. Cell-free DNA may contain circulating tumor DNA (ctDNA), i.e., tumor DNA circulating freely in the blood of a cancer patient or circulating fetal DNA (if the subject is a pregnant female). cfDNA can be obtained by centrifuging whole blood to remove all cells, and then isolating the DNA from the remaining plasma or serum. Such methods are well known (see, e.g., Lo et al, Am J Hum Genet 1998; 62:768-75). Circulating cell-free DNA can be double-stranded or single-stranded. This term is intended to encompass free DNA molecules that are circulating in the bloodstream as well as DNA molecules that are present in extra-cellular vesicles (such as exosomes) that are circulating in the bloodstream.

As used herein, the term "circulating tumor DNA" (or "ctDNA") is tumor-derived DNA that is circulating in the peripheral blood of a patient. ctDNA is of tumor origin and originates directly from the tumor or from circulating tumor cells (CTCs), which are viable, intact tumor cells that shed from primary tumors and enter the bloodstream or lymphatic system. The precise mechanism of ctDNA release is unclear, although it is postulated to involve apoptosis and necrosis from dying cells, or active release from viable tumor cells. ctDNA can be highly fragmented and in some cases can have a mean fragment size about 100-250 bp, e.g., 150 to 200 bp long. The amount of ctDNA in a sample of circulating cell-free DNA isolated from a cancer patient varies greatly: typical samples contain less than 10% ctDNA, although many samples have less than 1% ctDNA and some samples have over 10% ctDNA. Molecules of ctDNA can be often identified because they contain tumorigenic mutations.

As used herein, the term "sequence variation" refers to the combination of a position and type of a sequence alteration. For example, a sequence variation can be referred to by the position of the variation and which type of substitution (e.g., G to A, G to T, G to C, A to G, etc. or insertion/deletion of a G, A, T or C, etc.) is present at the position. A sequence variation may be a substitution, deletion, insertion or rearrangement of one or more nucleotides. In the context of the present method, a sequence variation can be generated by a genetic variation.

As used herein, the term "genetic variation" refers to a variation (e.g., a nucleotide substitution, an indel or a rearrangement) that is present or deemed as being likely to be present in a nucleic acid sample. A genetic variation can be from any source. For example, a genetic variation can be generated by a mutation (e.g., a somatic mutation), an organ transplant or pregnancy. If sequence variation is called as a genetic variation, the call indicates that the sample likely contains the variation; in some cases a "call" can be incorrect. In many cases, the term "genetic variation" can be replaced by the term "mutation". For example, if the method is being uses to detect sequence variations that are associated with cancer or other diseases that are caused by mutations, then "genetic variation" can be replaced by the term "mutation".

The term "amplicon" refers to a region of a genome that has been amplified by PCR. The number and sequences of a plurality of amplified regions should be the same as the number and sequences of the resulting amplicons. Thus, the terms "amplified regions" and "amplicons" can refer to the same thing.

The terms "tiled" and "tile across" refers to a set of primers that have complementary sites that are distributed across a region. All or most (e.g., at least 80% or at least 90%) of intervals between binding sites for a set of primers that are tiled across a region may be in the region of 20 to 200 nucleotides, where the average interval may be in the range of 40-150 nucleotides (excluding intervals that contain repetitive sequences).

As used herein, the term "value" refers to a number, letter, word (e.g., "high", "medium" or "low") or descriptor (e.g., "+++" or "++"). A value can contain one component (e.g., a single number) or more than one component, depending on how a value is analyzed.

Other definitions of terms may appear throughout the specification.

## DETAILED DESCRIPTION

Before the various embodiments are described, it is to be understood that the teachings of this disclosure are not limited to the particular embodiments described, and as such can, of course, vary. It is also to be understood that the terminology used herein is for the purpose of describing particular embodiments only, and is not intended to be limiting, since the scope of the present teachings will be limited only by the appended claims.

The section headings used herein are for organizational purposes only and are not to be construed as limiting the subject matter described in any way. While the present teachings are described in conjunction with various embodiments, it is not intended that the present teachings be limited to such embodiments. On the contrary, the present teachings encompass various alternatives, modifications, and equivalents, as will be appreciated by those of skill in the art.

The citation of any publication is for its disclosure prior to the filing date and should not be construed as an admission that the present claims are not entitled to antedate such publication by virtue of prior invention. Further, the dates of publication provided can be different from the actual publication dates which can need to be independently confirmed.

As will be apparent to those of skill in the art upon reading this disclosure, each of the individual embodiments described and illustrated herein has discrete components and features which can be readily separated from or combined with the features of any of the other several embodiments without departing from the scope or spirit of the present teachings. Any recited method can be carried out in the order of events recited or in any other order which is logically possible.

Numeric ranges are inclusive of the numbers defining the range. Unless otherwise indicated, nucleic acids are written left to right in 5' to 3' orientation; amino acid sequences are written left to right in amino to carboxy orientation, respectively.

It must be noted that as used herein and in the appended claims, the singular forms "a", "an", and "the" include plural referents unless the context clearly dictates otherwise. For example, the term "a primer" refers to one or more primers, i.e., a single primer and multiple primers. It is further noted that the claims can be drafted to exclude any optional element. As such, this statement is intended to serve as antecedent basis for use of such exclusive terminology as "solely," "only" and the like in connection with the recitation of claim elements, or use of a "negative" limitation.

As noted above, the present method provides a way to quantify DNA gene fusion molecules in a sample of cfDNA in a single assay. The reagent mix used in the method comprises two subsets of primers. The first subset of primers provides a way to identify fusion molecules, without knowing the fusion junction of the molecules beforehand and, in some cases, without knowing exactly which genes are fused. The second subset of primers provides a way to quantify the fusion molecules identified in the first component of the method.

In implementing the present method, it is important to note that fusion molecules are typically in a minority in the cfDNA. Specifically, because cfDNA is a mixture of normal DNA (released from normal, non-cancerous cells) and DNA that has been released from cancerous cells, the majority of fragments that are from the first or second regions of interest are typically not fusion molecules. For example, in most cfDNA samples from patients that have a cancer associated with a fusion between a first region and a second region, up to 90% of the fragment molecules corresponding to first region or second region will not be linked to the other region. Only DNA that has been released from the cancer cells (which typically represents up to 10% of the cfDNA, although sometimes more) contains the fusion molecules. The allelic fraction (i.e., the percentage of molecules that contain both sequences from the first and second regions, relative to molecules that contain the same sequences but not fused, is typically less than 10% ctDNA, although many samples have less than 1% ctDNA.

In general terms, the first subset of primers target regions that are usually unlinked or too far apart on a chromosome for an amplification product to be produced by PCR unless there is a genomic rearrangement. In many embodiments, the first subset of primers comprises a pool of at least 20 forward primers that tile across a first region of interest and a pool of at least 20 reverse primers that tile across a second region of interest, wherein the first and second regions of interest are different and either on different chromosomes or on the same chromosome and distanced by at least 1kb, at least 5kb or at least 10kb. If a genomic rearrangement event occurs, such as a gene fusion event, the two regions of interest are brought into proximity and at least one pair of the fusion-specific forward and reverse primers are sufficiently close to each other to produce an amplification product in a PCR. The sequence of the amplification product is then determined and the fusion junction, in some cases, which genes have been fused can be identified.

The first region of interest and the second region of interest should be on different chromosomes or sufficiently distanced in the reference genome so that no amplification products are expected unless there is a rearrangement in which the first region of interest and the second region of interest become closely linked to one another. In some embodiments, the first and second regions of interest should be on different chromosomes in the reference genome, or distanced by at least 10kb, at least 50kb, or at least 100kb if those regions are on the same chromosome in the reference genome. In embodiments in which cfDNA isolated from blood is analysed, the distance between the first and second regions of interest can be much shorter, e.g., at least 1kb or at least 5kb, because cfDNA is heavily fragmented (having a median size that is well below 1 kb, e.g., in the range of 50 bp to 500 bp) and, as such, no amplification products would be expected if the first and second regions are 1 kb or 5 kb apart.

The fusion-specific primers can be multiplexed in such as way that a variety of different fusions can be identified. For example, in some embodiments, the reaction mix may comprise i. multiple (e.g., 2, 3, 4, 5, 6 or up to 10 or more) sets of at least 20 fusion-specific forward primers, wherein within each set the fusion-specific forward primers tile across the same strand of a region in a reference human genome, and wherein each set targets a different kinase gene (e.g., RET, BRAF, NTRK1, NTRK3, ALK and ROS1, etc.), for example, and ii. multiple sets of at least 20 fusion-specific reverse primers, wherein within each set the fusion-specific reverse primers tile across the same strand in a different region of the reference human genome, wherein each set targets a fusion partner for the kinase genes targeted by the forward primers. In these embodiments, fusions can be identified and quantified without even knowing which genes have been fused beforehand.

The fusion identification method summarized above is described in greater detail in PCT/GB2018/051688, filed on June 18, 2018, and GB1709675.1, filed on June 16, 2017.

For example, PCT/GB2018/051688 describes which fusions can be identified, multiplexing strategies, how primers can be designed, how many primers can be tiled across a region, the density of the tiling, how long the regions are, which genes the primers hybridize to, barcoding strategies, PCR conditions, sample preparation, sequencing strategies and various definitions, etc.

Exemplary workflows for how the fusion-specific primers can be used to identify fusion molecules are shown in Figs. 7 and 8.

The second component of the method employs primers that target a reference region in the genome. A reference region is different to the first and second regions of interest and is not expected to increase or decrease in copy number between samples. Except for the sex chromosomes, repetitive sequences, duplicated sequences and sequence that are known to vary in copy number, a significant portion of the human genome could be considered a reference region. The reference primers are included in the same reaction mix as the fusion-specific primers.

In some embodiments, the present method comprises combining a test sample comprising cell-free DNA (cfDNA) obtained from the bloodstream of a human subject with a set of primers and a thermostable polymerase to produce a reaction mix, wherein the set of primers comprises at least 20 fusion-specific forward primers (as summarized above) and at least 20 fusion-specific reverse primers (as summarized above), and one or plurality of reference primer pairs.

The method may be performed using a single reference primer pair. However, in some embodiments, the method is performed using a plurality of primer pairs. If a single reference primer pair is used, then the region amplified by the reference primer pair is in the range of 40 bp to 160 bp (e.g., 80 bp to 120 bp) and should have a GC content of 25%-75%, which is similar to that expected for an "average" amplicon corresponding to the fusion. If a plurality of reference primer pairs is used, then each reference primer pair should amplify a different reference region of the genome, and the regions amplified by the reference primer pairs should be of different lengths, each in the range of 40 bp to 160 bp. For example, in some cases, the lengths of the regions amplified by the reference primer pairs should differ from each other by at least 3 nucleotides, at least 4 nucleotides, at least 5 nucleotides or at least 10 nucleotides, such that the lengths of the regions amplified by the reference primer pairs (or the amplicons amplified by the same) are distributed in the range of 40 bp to 160 bp. As noted below, if a sample that has a different fragmentation profile is used (e.g., intact genomic DNA, or DNA isolated from FFPE samples, urine or CSF, etc.) this range may be adjusted accordingly. Additionally, the GC content of the regions amplified by the reference primer pairs may vary from region to region in the range of 25% to 75%. For example, in some embodiments the GC content of the regions amplified by the reference primer pairs may differ from each other by at least 2%, at least 3%, at least 4% or at least 5%. If a plurality of primer pairs is used, then in some embodiments the set of primers used in the method may comprise at least 5 or at least 10 reference primer pairs. In some embodiments, the set of primers may contain up to 100 reference primer pairs, although a primer set may comprise 10 to 30 reference primer pairs in many cases.

After the reaction mix has been made, the reaction mix is thermocycled to produce PCR products. The polymerase used in the method can be any suitable thermostable polymerase such as Taq polymerase, VENT, and Phusion polymerase, etc., and, as would be apparent, necessary cofactors (e.g., $Mg^{2+}$, salt, and a buffering agent) should be present in the reaction. The thermocycling conditions and temperatures conditions may be readily adapted from to those used for PCR, e.g., may involve 10-40 cycles of that include a denaturation step at a temperature of over 90 °C, e.g., at about 95 °C, an annealing step at a temperature in the range of 50 °C to 75 °C, and an extension step at a temperature of 70-75 °C. Two step cycling may also be used.

Depending on a variety of factors, (e.g., how many reference primer pairs are used and the density of tiling of the fusion-specific primers) thermocycling the reaction mix may result in a reaction product comprising as few as two amplicons (one corresponding to a fusion event and the other corresponding to a reference region). In many embodiments, however, the reaction product may comprise a plurality of amplicons. In these embodiments, the gene fusion may be represented by several overlapping amplicons (e.g., 2, 3 or 4 amplicons, depending on the density of the fusion-specific primers and other factors). As would be apparent, the number of a reference amplicons should correspond to the number of the reference primer pairs used. As such, if the reaction mix contains 15 reference primer pairs, then the product should contain 15 reference amplicons that correspond to the reference regions. As such, thermocycling the reaction mix should result in i. one or more reference amplicon that are produced by the reference primer pairs (depending on how many pairs of primers are used), and ii. one or more fusion amplicons that are produced by the fusion-specific primers using fusion molecules in the cfDNA as a template. As would be apparent, the fusion molecules correspond to a genomic rearrangement that fuses the first region with the second region in at least some cells of the subject.

Because the sequence of the human genome (and other genomes) is known, primer sets can be readily designed before use. In some embodiments, the primers used may have 5' tails that allow the amplification products to be re-amplified prior to sequencing. For example, in some embodiments, the fusion-specific forward primers and one primer from each reference primer pair may have a first tail, and the fusion-specific reverse primers and the primer from each reference primer pair may have a second 5' tail, thereby allowing the amplification products to be amplified using primers that have the same sequence as the tail.

Any tail and/or universal primer can include other informational sequences such as sample barcodes, index sequences, random sequences and/or replicate barcodes, as desired. As would be apparent, the tails of the primers and/or the universal primers may be compatible with use in the next generation sequencing platform used for sequence analysis, e.g., Illumina's reversible terminator method, Roche's pyrosequencing method (454), Life Technologies' sequencing by ligation (the SOLiD platform), Life Technologies' Ion Torrent platform or Pacific Biosciences' fluorescent base-cleavage method. Examples of such methods are described in the following references: Margulies et al (Nature 2005 437: 376-80); Ronaghi et al (Analytical Biochemistry 1996 242: 84-9); Shendure (Science 2005 309: 1728); Imelfort et al (Brief Bioinform. 2009 10:609-18); Fox et al (Methods Mol Biol. 2009;553:79-108); Appleby et al (Methods Mol Biol. 2009;513:19-39) English (PLoS One. 2012 7: e47768) and Morozova (Genomics. 2008 92:255-64). Nanopore sequencing may be used in some embodiments.

Next, the amplicons produced by thermocycling the reaction, or amplification products thereof (if the amplicons are re-amplified by universal primers that hybridize to 5' tails in the primers) are sequenced to produce sequence reads. The sequencing step may be done using any convenient next generation sequencing method and may result in at least at least 100,000, at least 500,000, at least 1M at least 10M at least 100M, at least 1B or at least 10B sequence reads per reaction. In some cases, the reads may be paired-end reads.

The sequence reads are then processed computationally. The initial processing steps may include identification of barcodes (including sample identifiers or replicate identifier sequences), and trimming reads to remove low quality or adaptor sequences. In addition, quality assessment metrics can be run to ensure that the dataset is of an acceptable quality.

After the sequence reads have undergone initial processing, they are analyzed to identify which reads correspond to a fusion, and which reads correspond to reference regions. Reads that correspond to a fusion should contain two sequences that are not next to each other in the reference genome, i.e., a first sequence from the first region and a second sequence from the second region, if the fusion-specific primers are designed to those regions. The reads that correspond to the reference regions can be identified because they are identical or near identical to a references sequence.

After the numbers of sequence reads corresponding to the fusion molecules and reference regions have been determined, the relative abundance of the fusion molecules can be determined by comparing the number of sequence reads corresponding to fusion molecules with the number of sequence reads corresponding to the reference region, to produce a ratio. The ratio may be expressed as a percentage in some cases. The ratio provides a way to compare the amount of fusion DNA across samples. In particular embodiments, two or more samples of cfDNA taken from the same patient at different time-points may be analysed to provide a ratio at each time point. The ratios can be compared to determine if there are any changes in the abundance of the fusion DNA over time. This embodiment of the method may comprise separately analysing a first test sample and a second test sample (where the first and second test samples are obtained from the same subject at different time points) using the present method, to obtain a first ratio indicating the abundance of the fusion molecules in the first sample and a second ratio indicating the abundance of the fusion molecules in the second test sample; and comparing the first and second ratios to determine if the abundance of the fusion molecules has changed over time. In some embodiments, the method may comprise separately analysing test samples obtained from the same subject on at least 3 different time points (e.g., 3, 4, or 5 or more time-points) using the present to obtain a time-course of ratios indicating the abundance of the fusion molecules in the test samples over time. The time-points may be spaced by days, weeks or months, for example.

In certain cases, the method may further involve comparing the time-course of ratios to the time course of an allele frequency of another mutation in the sample, where the mutation is a single nucleotide variation or in-del. In these embodiments, any changes observed in the abundance of the fusion DNA may be mirrored by a change in the abundance of a secondary mutation. If a change in the abundance of the fusion DNA is accompanied by a change in the abundance of a secondary mutation, then one can be more confident of the chance.

The method may be used for a variety of different purposes. For example, the method may be used to monitor disease progression, to determine whether a treatment has been effective, to determine whether a switch in treatment is appropriate, to confirm that a patient has gone into remission and/or to determine if a cancer has recurred. In some embodiments, the subject may have cancer. These embodiments may be used to determine if a treatment is working since, as noted above, the abundance of a fusion DNA in the sample should decrease relative to the reference sequence over time if a treatment is successful.

In some cases, cfDNA from a cancer patient may be tested using the present method to reveal that the patient has cfDNA containing a particular fusion. On the basis of this knowledge, the patient may receive a treatment that targets the fusion. For example, the analysis indicates that the patient's cancer is associate with a tyrosine kinase fusion, then the patient may be treated with a tyrosine kinase inhibitor such as crizotinib (Xalkori), ceritinib (Zykadia), alectinib (Alecensa) or brigatinib (Alunbrig), entrectinib (RXDX-101), lorlatinib (PF-06463922), ropotrectinib (TPX-0005), DS-6051b, ensartinib, larotrectinib (VITRAKVI) or cabozantinib (Cometriq, Cabometyx). After a period of a few days, weeks or months, cfDNA from a cancer patient may be tested again using the present method to reveal if the fusion molecules have increased or decreased over time. As noted above, a decrease in the amount of fusion molecules indicates that the therapy is working and should be continued. An increase in the amount of fusion molecules indicates that the therapy is not working and should be discontinued or altered if better options are available.

As noted above, in some embodiments, the method may employ a plurality of reference primer pairs that amplify different reference reasons. In these embodiments, the relative abundance of the fusion molecules may be quantified by comparing the number of sequence reads corresponding to fusion molecules with the median number of sequence reads corresponding to at least some (e.g., at least 5 or at least 10) of the reference regions. This embodiment of the method may involve eliminating the sequence reads corresponding to some (e.g., up to 5 or 10, but not all) of the reference regions. In these embodiments, the relative abundance of the fusion molecules may be quantified by: i. eliminating sequence reads corresponding to one or more of the reference sequences and ii. comparing the number of sequence reads corresponding to fusion molecules with the median number of remaining sequence reads that correspond to the reference regions. Sequence reads corresponding to a particular region may be eliminated for a variety of different reasons. For example, in some cases, for unknown reasons some regions do not amplify very efficiently or are more susceptible to sample-to-sample variations (e.g., impurities in the sample). These "outlier" regions can be identified over time and eliminated from the analysis (i.e., computationally), if necessary. As such, in some embodiments, sequence reads corresponding to outlier reference regions are eliminated. In other embodiments, the method may comprise eliminating sequence reads corresponding to reference regions that are not closely matched with the one or more fusion amplicons in GC content and/or length. In these embodiments, the sequence of a fusion amplicon may be determined and one or more of the best matched (in terms of length and/or G/C content) reference regions may be selected for the remaining analysis. For example, if a fusion amplicon is 150 bp in length and has a G/C content of 50%, then the sequence reads corresponding to reference regions that have the most similar length and/or G/C content can be selected (computationally) from future analysis. The other reads can be eliminated. In some embodiments, sequence reads that correspond to reference regions that have lengths that are +/-20%, +/-10% or +/-5% of the fusion amplicons, can be selected. In some embodiments, certain reference regions may be excluded as they are likely to have copy number changes in the cancer type being tested. In other embodiments a separate analysis may be performed in order to identify references regions that have a change in copy number in the patient of interest. Any reference regions likely to contain copy number changes may be eliminated. Likewise, in some embodiments, sequence reads that correspond to reference regions that have a G/C content that is +/-20%, +/-10% or +/-5% of the fusion amplicons, can be selected.

In some embodiments, the method can be done using replicate samples. In these embodiments, a sample of cfDNA from each time-point can be split into two, three or four or more replicates which are independently analysed using the present method. Each replicate should produce a ratio indicating the amount of fusion DNA in the cfDNA. In these embodiments, for each replicate, the ratios can be combined or averaged to provide, e.g., a mean or average ratio. Ratios that have a lower variability provide a high confidence whereas ratios that have a higher variability provide a lower confidence. As such, the variability of the ratios at each time-point can be used to provide a confidence that a change in the amount of fusion DNA has occurred over time. In these embodiments, the method may comprise calculating the variability between the ratios for the replicate samples at each time point, and using the variability at each timepoint to determine the confidence that a difference of ratios between different time points reflects change in abundance of the fusion molecules over time. For example, in some embodiments the method may comprise calculating a standard error for the replicate samples at each time point, and using the standard errors to estimate the significance of a difference in ratios across different time points for the same patient. This concept is illustrated in Fig. 9.

It has been noted that multiple overlapping amplicons corresponding to a single gene fusion may be produced in some experiments. This may be caused by, for example, some fragments having binding sites for two forward primers and one binding for a reverse primer. In this example, two amplicons representing the fusion may be produced. In these embodiments, the presence of two fusion amplicons (which can be determined by analysing the sequence reads) may increase the confidence that the sample contains fusion molecules. In these embodiments, the method may further comprise determining whether there are multiple fusion amplicons corresponding to the genomic rearrangement. If identified, the presence of multiple fusion amplicons corresponding to the genomic rearrangement increases the confidence that at least some of the cells of the subject comprise the genomic rearrangement. In addition, sequence reads corresponding to the different fusion amplicons can be analysed separately or together. In these embodiments, the method may be done by comparing the number of sequence reads corresponding to one or more of the reference sequences to i. the number of sequence reads corresponding to each of the multiple fusion amplicons to produce multiple ratio, or ii. all of the multiple fusion amplicons to produce a single ratio. In some embodiments, if each fusion amplicon is analysed separately the can be compared to different references sequences as described earlier. The results can then either be combined or the best fusion, for example the one with the most reads kept. In particular embodiments, one could separately normalise the sequence reads for each amplicon to the reference regions and then take the median or mean of each of the normalised values. This would provide an "average" normalised value which could plotted over time. In another example, one could separately normalise the sequence reads for each amplicon to the reference regions and also analyse how the different amplicons change over time. In these embodiments, if all of the different fusion amplicons show the same pattern then the results should be more trustworthy. In another example one could add up all the reads to the different fusion amplicons and then normalise to the reference regions.

The at least 20 fusion-specific forward primers may comprise at least 50 or at least 100 different forward primers and, independently, the at least 20 fusion-specific reverse primers may comprise at least 50 or at least 100 different reverse primers. The average interval between adjacent binding sites for the forward primers in the first region should no more than 100 bases and, in some embodiments, the intervals are all in the range of 20 to 100 bases (e.g., 50 to 100 bases). Likewise the average interval between adjacent binding sites for the reverse primers in the second region should no more than 100 bases and, in some embodiments, the intervals are all in the range of 20 to 100 bases (e.g., 50 to 100 bases), except for intervals that contain repetitive sequence. These intervals may be increased in samples in which the DNA is more intact.

Several gene fusions that are thought to cause cancer have already been identified and may be targeted by the fusion-specific primers. As such, in some embodiments, the first region to which the forward primers bind may be selected from the group consisting of ROS1, ALK, EML4, BCR, ABL, TCF3, PBX1, ETV6, RUNX1, MLL, AF4, SIL, TALI, RET, NTRK1, PAX8, PPARG, MECT1, MAML2, TFE3, TFEB, BRD4, NUT, ETV6, NTRK3, TMPRSS2, NKRT2 and ERG. In some embodiments, the fusion-specific forward primers may hybridize to ALK, RET, NTRK1, ROS1, BRAF, EGFR, NRG1 or MET. Possible fusion partners for these genes are numerous. For example, if the forward primers hybridize to the ALK gene, then the reverse primers may hybridize to EML4, STRN, KIF5B and/or TFG. Likewise, if the forward primers hybridize to the ROS1 gene, then the reverse primers may hybridize to CD74, SLC34A2, SDC4, TPM3 and/or EZR. In some embodiments, the fusion-specific primers may target any one or more of the following fusions: CD74-ROS1, SLC34A2-ROS1, SDC4-ROS1, EZR-ROS1, GOPC-ROS1, LRIG3-ROS1, TPM3-ROS1, PPFIBP1-ROS1, EML4-ALK, BCR-ABL, TCF3-PBX1, ETV6-RUNX1, MLL-AF4, SIL-TAL1, RET-NTRK1, PAX8-PPARG, MECT1-MAML2, TFE3-TFEB, BRD4-NUT, ETV6-NTRK3, TMPRSS2-ERG, TPM3-NTRK1, SQSTM1-NTRK1, CD74-NTRK1, MPRIP-NTRK1 and TRIM24-NTRK2.

As may be apparent, the method may be multiplexed such that several different gene fusions can be targeted in the assay. Illustrated by example, in some embodiments, the primer set may comprise a first subset of at least 20 fusion-specific forward primers, wherein this subset of primers specifically hybridize to the same strand of a first region in a reference human genome (e.g., the ALK gene), a second subset of at least 20 fusion-specific forward primers, wherein this set of primers specifically hybridize to the same strand of a second region in the reference human genome (e.g., the ROS1 gene) and, optionally, further sets of at least 20 fusion-specific forward primers that specifically hybridize to the same strand of other regions. In these embodiments, the primer set may additionally contain multiple sets of at least 20 fusion-specific reverse primers, wherein each set of fusion-specific reverse primers specifically hybridize to the same strand in potential fusion partners for the genes targeted by the forward primers. For example, if kinase genes (e.g., ALK and ROS1) are targeted by the forward primers in one reaction, then the reverse primers used in the reaction can target at least two, at least three, at least four, at least five, at least six or all of EML4, STRN, KIF5B, TFG, CD74, SLC34A2, SDC4, TPM3 and/or EZR.

Reaction mixes used in the present method may comprise hundreds or even thousands (e.g., at least 200, at least 500, at least 1,000, at least 5,000 or at least 10,000) of different primers where the primers comprise multiple sets of the primers, each containing at least 20, at least 40 or more (e.g., up to 500 or 1,000) primers although some fusions may be detected with sets of primers that contain less primers. The different sets of primers hybridize to different regions of the human genome where the different regions are unlinked or separated by at least 10 kb (e.g., at least 100 kb). For example, a single reaction may comprise at least 40 primers that are tiled across a strand of the ALK gene (which encodes a kinase), at least 300 hundred primers that are tiled across a strand of the EML4 gene (which is a potential fusion partner for ALK) and at least 100 primers that are tiled across a strand of the STRN gene (which is another potential fusion partner for ALK). The same or a different reaction may also comprise at least 100 primers that are tiled across a strand of the RET gene (which encodes a kinase). The reaction containing the RET primers may also comprise at least 20 primers that are tiled across a strand of the TRIM33 gene (a potential fusion partner for RET), at least 40 primers that are tiled across a strand of the CCDC6 gene (another potential fusion partner for RET), at least 100 primers that are tiled across a strand of the KIF5B gene (another potential fusion partner for RET) as well as primers that are tiled across a strand of the NCOA4 gene (another fusion partner for RET). In another embodiment, a single reaction may comprise at least 150 primers that are tiled across a strand of the ROS1 gene (which encodes a kinase), at least 50 primers that are tiled across a strand of the SLC34A2 gene (a potential fusion partner for ROS1), at least 40 primers that are tiled across a strand of the CD74 gene (another potential fusion partner for ROS1) and at least 50 primers that are tiled across a strand of the SDC4 gene (another potential fusion partner for ROS1). The same or a different reaction may also comprise at least 50 primers that are tiled across a strand of the NTRK1 gene (which encodes a kinase). The reaction containing the NTRK1 primers may also comprise at least 100 primers that are tiled across a strand of the SQSTM1 gene (a potential fusion partner for NTRK1).

Within each set of primers, the primers hybridize to the same strand of a region of the human genome. As noted above, using cfDNA as a template, PCR reactions that contain these primers only produce amplicons if there are fusion molecules in the sample; no or few spurious "side amplicons", i.e., amplicons produced by non-specific binding of the primers to the template, are produced. This may be unexpected given the complexity of the reaction. Without wishing to be bound to any specific theory, it is thought that highly multiplexed PCR reactions using cfDNA produce less spurious amplicons because cfDNA is so fragmented (relative to typical samples that contain human DNA in which the average fragment size may be at least 10kb, e.g., 50 kb to 500 kb). The reasons for this are unclear. However, again without wishing to be bound to any specific theory, it is theorized that in each cycle of the PCR, any primer extension products generated by non-specific priming events should be very short (e.g., averaging 30 to 150 bases) because the template molecules in cfDNA are very short. If an intact genome is amplified using the same primers, then any primer extension products generated by non-specific priming may be several hundred bases or even kilobases in length. Primer extension products generated by non-specific priming accumulate in the sample and cause more non-specific priming events. Due to this the probability of producing spurious amplicons should be much higher with an intact genome is used as a template. In other words, non-specific primer extension products are much longer when a sample comprising intact genomic DNA is used, which, itself, generates more potential for non-specific primer extension products in each cycle. Because these products accumulate, many more spurious amplicons are produced when an intact genome is used, rather than cfDNA. Because PCR reactions using cfDNA as a template should, in theory, produce much less (i.e., shorter) non-specific primer extension products than PCR reactions using intact human genomic DNA as a template, it is thought that a PCR reaction that use cfDNA as a template may be able to accommodate many more primers (potentially as many as 10-fold or 100-fold more primers) than PCR reactions that use an intact genome as a template.

Further, as noted above, the present method, when it is practiced using cfDNA as a template, is believed to benefit from analysis of replicates in which a sample is split and the replicates are analyzed in parallel using the same analysis method. If a fusion is identified in two or more replicates, then it is almost certainly in the sample. Without wishing to be bound to any specific theory, it is thought that cfDNA is fragmented relatively randomly with some bias potentially towards fragmenting either side of nucleosome binding sites, and any products that are produced by extension of primers that have hybridized to the correct sequence in a kinase or fusion partner gene should, in theory, have sequence at the 3' end that corresponds to the end of the fragment from which it was copied (i.e., the fragmentation break point). Because the fragmentation breakpoints are very variable, the 3' end of some of the primer extension products could, in theory, hybridize to another site in the genome. This is not necessarily problematic unless the 3' end of a primer extension product produced by extension of a kinase-specific primer is an exact match for a potential fusion partner (or vice versa) or a region with close similarity to a potential fusion partner (or vice versa). If this happens, then the primer extension product (which contains part of a kinase gene) could, in theory, be extended using the potential fusion partner gene or similar region as a template (or vice versa). This fusion molecule, which may have a kinase sequence at one end and a potential fusion partner sequence at the other could be amplified in future PCR cycles, thereby producing a fusion amplicon that is really a PCR artefact. Replicate samples solve this problem because fragmentation occurs at random sites and which strands get amplified relies on random hybridizations events. As such, in the unlikely event that a rogue fusion molecule is detected in one replicate, the fusion cannot be called unless it is found in a replicate. Replicates should not produce the same PCR artefacts and therefore, in some embodiments before a sample can be identified as having a particular gene fusion, fusion molecules derived from the gene fusion and same breakpoint sequence should be identified in multiple replicates.

Also provided is a method of detecting a genomic fusion event. This method may comprise: (a) combining a test sample comprising cell-free DNA (cfDNA) from the bloodstream of a human subject with at least 20 forward primers, at least 20 reverse primers, and a polymerase to produce a reaction mix, wherein: i. the forward primers specifically hybridize to the same strand of a first region in a reference human genome, wherein the average interval between adjacent binding sites for the forward primers in the first region is no more than 100 bases; or i. the forward primers specifically hybridize to the same strand of a first region in a reference human genome, wherein the interval between adjacent binding sites for the forward primers in the first region is no more than 100 bases, excluding intervals that contain repetitive sequences; ii. the reverse primers specifically hybridize to the same strand in a second region of the reference human genome, wherein the interval between adjacent binding sites for the reverse primers in the second region is no more than 100 bases; or ii. the forward primers specifically hybridize to the same strand of a first region in a reference human genome, wherein the interval between adjacent binding sites for the forward primers in the first region is no more than 100 bases, excluding intervals that contain repetitive sequences, and iii. the first and second regions are on different chromosomes or are on the same chromosome but spaced apart by at least 10 kb; and (b) thermocycling the reaction mix to produce PCR products only if the patient has a tumor comprising a genomic rearrangement that fuses the first region with the second region. In these embodiments, the primers are tile across their respective regions such that all or most (e.g., at least 80% or at least 90%) of intervals between binding sites for a set of primers that are tiled across a region may be in the region of 20 to 200 nucleotides, where the average interval may be in the range of 40-150 nucleotides (excluding intervals that contain repetitive sequences).

In these embodiments, the first region may be in the ALK gene and the second region may be in the EML4 or STRN genes, the first region may be in the RET gene and the second region may be in the TRIM33, CCDC6 or KIF5B genes, the first region may be in the ROS1 gene and the second region may be in the SLC34A2, CD74 or SDC4 genes, or the first region may be in the NTRK1 gene and the second region may be in the SQSTM1 gene.

Also provided is another method for detecting a genomic fusion event. This method may comprise: (a) combining a test sample comprising cell-free DNA (cfDNA) from the bloodstream of a human subject with a first set of at least 20 primers, a second set of at least 20 primers, a third set of at least 20 primers, and, optionally, a third set of at least 20 primers and a polymerase to produce a reaction mix, wherein: the first set of primers specifically hybridize to the same strand of a kinase gene; the second set of primers specifically hybridize to the same strand of a first potential fusion partner for the kinase gene; the second set of primers specifically hybridize to the same strand of a second potential fusion partner for the kinase gene; the second set of primers specifically hybridize to the same strand of a third potential fusion partner for the kinase gene; and the kinase gene, and the first, second and third fusion partners for the kinase gene are on different chromosomes or are on the same chromosome but spaced apart by at least 10 kb in a reference human genome that does not have a genomic rearrangement that fuses the kinase region with the first, second or third fusion partner; and (b) thermocycling the reaction mix to produce PCR products only if there is a genomic rearrangement that fuses the kinase region with the first, second or, optionally, the third fusion partner in a tumor in the subject.

In these embodiments, the kinase gene may be ALK and the first and second fusion partners may be selected from EML4 and STRN, the kinase gene may be RET first, second and third fusion partners may be selected TRIM33, CCDC6 and KIF5B genes, the kinase gene may be ROS1 and the first, second and third fusion partners may be selected from SLC34A2, CD74 and SDC4 genes, for example.

A highly multiplexed polymerase chain reaction for detecting gene fusion events is also provided This method may comprise (a) combining a test sample comprising cell-free DNA (cfDNA) from the bloodstream of a human subject with a pool of at least 500, at least 600, or at least 700, at least 800, at least 900, or at least 1000 primers and a polymerase to produce a reaction mix, wherein the primers only amplify a sequence if there is a genomic rearrangement that fuses the kinase region with a fusion partner in a tumor of the subject, and (b) thermocycling the reaction mix to produce PCR products. PCR products should only be produced if there is a genomic rearrangement that fuses the kinase region (e.g., ALK, RET, RO1 or NTRK1) with a fusion partner in a tumor of the subject.

A method for detecting fusion events using replicate PCR reactions is also provided. This method may comprise: (a) splitting a test sample comprising cell-free DNA (cfDNA) from the bloodstream of a human subject into a first replicate and a second replicate; (b) combining the first and second replicates with the same primers and polymerase to produce a first reaction mix and a second reaction mix, wherein the primers in the first reaction mix and a second reaction mix both comprise a first set of primers and a second set of primers, wherein: i. the primers of the first set specifically hybridize to the same strand of a first region in a reference human genome; ii. the primers of the second set specifically hybridize to the same strand of a second region in the reference human genome; wherein the first and second regions are on different chromosomes or are on the same chromosome but spaced apart by at least 10 kb; (c) thermocycling the first and second reaction mixes to produce first and second PCR products; (d) independently identifying, by sequencing, whether the same fusion molecule exists in the first and second PCR products; wherein a fusion molecule that exists in both the first and second fusion products indicates that the human subject has a tumor which comprises a genomic rearrangement that fuses the first region with the second region, and wherein a fusion molecule that is exists in neither or only one of the first and second fusion products indicates that the human subject does not have a tumor comprising the genomic rearrangement

In these embodiments, the first region may be in the ALK gene and the second region may be in the EML4 or STRN genes, the first region may be in the RET gene and the second region may be in the TRIM33, CCDC6 or KIF5B genes, the first region may be in the ROS1 gene and the second region may be in the SLC34A2, CD74 or SDC4 genes, or the first region may be in the NTRK1 gene and the second region may be in the SQSTM1 gene.

In some embodiments, the method may comprise providing a report indicating the relative abundance of the fusion molecules at different timepoints. This could be conveniently illustrated as a graph (such as that shown in Fig. 9) in some embodiments. In addition, a report may provide options for approved (e.g., FDA approved) therapies if there is a change in abundance that indicates that such a therapy would be appropriate, to determine if a therapy is working or to determine if a switch in therapy is appropriate.

In some embodiments, the report may be in an electronic form, and the method comprises forwarding the report to a remote location, e.g., to a doctor or other medical professional to help identify a suitable course of action, e.g., to identify a suitable therapy for the subject. The report may be used along with other metrics to determine whether the subject may be susceptible to immune checkpoint inhibition.

In any embodiment, a report can be forwarded to a "remote location", where "remote location," means a location other than the location at which the sequences are analyzed. For example, a remote location could be another location (e.g., office, lab, etc.) in the same city, another location in a different city, another location in a different state, another location in a different country, etc. As such, when one item is indicated as being "remote" from another, what is meant is that the two items can be in the same room but separated, or at least in different rooms or different buildings, and can be at least one mile, ten miles, or at least one hundred miles apart. "Communicating" information references transmitting the data representing that information as electrical signals over a suitable communication channel (e.g., a private or public network). "Forwarding" an item refers to any means of getting that item from one location to the next, whether by physically transporting that item or otherwise (where that is possible) and includes, at least in the case of data, physically transporting a medium carrying the data or communicating the data. Examples of communicating media include radio or infra-red transmission channels as well as a network connection to another computer or networked device, and the internet, including email transmissions and information recorded on websites and the like. In certain embodiments, the report may be analyzed by an MD or other qualified medical professional, and a report based on the results of the analysis of the sequences may be forwarded to the patient from which the sample was obtained.

In computer-related embodiments, a system may include a computer containing a processor, a storage component (i.e., memory), a display component, and other components typically present in general purpose computers. The storage component stores information accessible by the processor, including instructions that may be executed by the processor and data that may be retrieved, manipulated or stored by the processor.

The storage component includes instructions for providing a score using the measurements described above as inputs. The computer processor is coupled to the storage component and configured to execute the instructions stored in the storage component in order to receive patient data and analyze patient data according to one or more algorithms. The display component may display information regarding the diagnosis of the patient.

The storage component may be of any type capable of storing information accessible by the processor, such as a hard-drive, memory card, ROM, RAM, DVD, CD-ROM, USB Flash drive, write-capable, and read-only memories. The processor may be any well-known processor, such as processors from Intel Corporation. Alternatively, the processor may be a dedicated controller such as an ASIC.

The instructions may be any set of instructions to be executed directly (such as machine code) or indirectly (such as scripts) by the processor. In that regard, the terms "instructions," "steps" and "programs" may be used interchangeably herein. The instructions may be stored in object code form for direct processing by the processor, or in any other computer language including scripts or collections of independent source code modules that are interpreted on demand or compiled in advance.

Data may be retrieved, stored or modified by the processor in accordance with the instructions. For instance, although the diagnostic system is not limited by any particular data structure, the data may be stored in computer registers, in a relational database as a table having a plurality of different fields and records, XML documents, or flat files. The data may also be formatted in any computer-readable format such as, but not limited to, binary values, ASCII or Unicode. Moreover, the data may comprise any information sufficient to identify the relevant information, such as numbers, descriptive text, proprietary codes, pointers, references to data stored in other memories (including other network locations) or information which is used by a function to calculate the relevant data.

The method described above may be readily adapted for use in other sample types (tumor tissue, CSF, urine, etc.). The method described above may be readily adapted for other types of rearrangements for example deletions or amplifications. With deletions for example, the two regions that may be brought together by the deletion may be tiled. In these cases, the tiling strategies and lengths of the reference regions may be adapted to the average length of the DNA fragments in those samples.

The present invention will now be further illustrate using a number of non-limiting examples.

## EXAMPLES

Aspects of the present teachings can be further understood in light of the following examples, which should not be construed as limiting the scope of the present teachings in any way.

Example 1: Detection of EML4-ALK Variant at a range of Allelic Fractions.

A custom cell free DNA reference standard containing an EML4-ALK fusion of sequence GAAGTTCCTATACTTTCTAGAGAATAGGAACTTC (SEQ ID NO: 9)_at an allelic fraction of 2.5% was obtained from Horizon Discoveries. This reference standard was diluted in sheared (average 188bp) human placental DNA (Bioline) to achieve allelic fractions of 1%, 0.5%, 0.25%, 0.125% and 0.0625%. Three samples were created at each allelic fraction.

Each sample was split into two replicates, each containing a total of 4000 input copies. PCR amplification was performed on two replicates using the ALK primer panel (table 1). Each PCR contained 25 uL DNA, 27.5 uL Platinum SuperFi 2x Master Mix (Invitrogen) and 2.5 uL of the ALK primer pool (for primer concentration see table 1). PCR cycling was followed using manufacturer' instructions. The PCR product was cleaned up using SPRIselect reagent (Beckman Coulter B23319) using the manufacturers protocol. DNA was eluted in 18 uL and a second PCR using Indexed illumina primers was performed. Each PCR contained 15 uL DNA, 17.5 uL Platinum SuperFi 2x Master Mix (Invitrogen) and 2.4 uL Indexed illumina primers. PCR Cycling was followed using manufactures instructions. The PCR product was cleaned up once using SPRIselect reagent (Beckman Coulter B23319) using the manufacturers protocol. Indexed samples from different replicates were pooled into a tube containing 10uL lOmM Tris-HCl pH 8. Samples were selected for 195-350 bp using a 2% Agarose Dye Free cassette and marker L on the Pippin Prep (Sage Science), following the manufacturer's instructions. Size selected DNA was quantified by qPCR using a KAPA Library quantification kit (KAPABIOSYSTEMS), following the manufacturer's instructions. Quantified libraries were sequenced on the NextSeq500 Illumina platform and data analysis was performed.

EML4-ALK enrichment using Selective PCR and Next generation sequencing (Figure 1). The EML4-ALK fusion variant was detected at all allelic fractions tested (Figure 2); illustrating that selective PCR consistently amplifies as little as 2.5 molecules of Fusion DNA as indicated by 100% detection at 0.0625% AF (4000 input copies). The sequence obtained by the selective PCR method matched the expected breakpoint (Figure 2A), indicating the selective nature of the method. Specificity of the method is at 100% with no additional Fusions detected in any of the samples tested and with no fusion calls being made in samples that don't contain Fusion DNA (0% AF). The median read depth of the EML4-ALK fusion (Figure 3), at a range of AFs, shows a decrease in reads obtained by selective PCR that correlates with a decrease in AF, indicating linear amplification of the gene fusion.

Example 2: Detection of CD74-ROS1 Variant

A synthetic gBlock containing a ROS1 fusion sequence (based on a sequence reported in the literature: Seki, Mizukami and Kohno, Biomolecules, 2015, 5, 2464-2476) was synthesized by IDT and was sheared using the covaris to achieve an average size of 150bp. The gBlock was added to sheared (average 188bp) human placental DNA (Bioline) to achieve an allelic fraction of 1%.

Each sample was split into two replicates, each containing a total of 4000 input copies. PCR amplification was performed on two of the replicates using the ROS1 primer panel (table 2). Each PCR contained 25 uL DNA, 27.5 uL Platinum SuperFi 2x Master Mix (Invitrogen) and 2.5 uL of the ROS1 primer pool (for primer concentration see table 2). PCR Cycling was followed using manufactures instructions. The PCR product was cleaned up using SPRIselect reagent (Beckman Coulter B23319) using the manufacturers protocol. DNA was eluted in 18 uL and a second PCR using Indexed illumina primers was performed. Each PCR contained 15 uL DNA, 17.5 uL Platinum SuperFi 2x Master Mix (Invitrogen) and 2.4 uL Indexed illumina primers. PCR Cycling was followed using manufactures instructions. The PCR product was cleaned up once using SPRIselect reagent (Beckman Coulter B23319) using the manufacturers protocol. Indexed samples from different replicates were pooled into a tube containing 10uL lOmM Tris-HCl pH 8. Samples were selected for 195-350 bp using a 2% Agarose Dye Free cassette and marker L on the Pippin Prep (Sage Science), following the manufacturer's instructions. Size selected DNA was quantified by qPCR using a KAPA Library quantification kit (KAPABIOSYSTEMS), following the manufacturer's instructions. Quantified libraries were sequenced on the NextSeq500 Illumina platform and data analysis was performed.

CD74-ROS1 enrichment using selective PCR and Sequencing on the NextSeq platform (Figure 4). The sequence of the ROS1-CD74 fusion breakpoint is known in the field (Seki, Mizukami and Kohno, Biomolecules, 2015, 5, 2464-2476) and was synthesised into a double stranded DNA fragment (Figure 5). The method detected the fusion breakpoint with two primer pairs, CD74_E6-I6_10/ROS1_I32_E33_414 and CD74_E6-I6_9/ROS1_I32_E33_414 (Figure 6A). The sequence read of both primer pairs matched that of the synthesised published CD74-ROS1 breakpoint (Figure 6B). The sequence read obtained for each primer pair is shown and is a 100% match with the published breakpoint; highlighting that the selective PCR method can amplify a fusion breakpoint with multiple primer combinations and can accurately identify the sequence of a fusion breakpoint.

Example 3: Detection of CD74-ROS1 Variant using sequential amplification

The same synthetic ROS1 fusion gBlock at 1% allelic fraction as was used in Example 2 was tested.

Each sample was split into two replicates, each containing a total of 4000 input copies. Linear amplification of the template was performed on two of the replicates using only the ROS1 forward primer panel. Each reaction contained 25 uL DNA, 27.5 uL Platinum SuperFi 2x Master Mix (Invitrogen) and 2.5 uL of the ROS1 forward primer pool. Cycling was followed using manufactures instructions. The PCR product was cleaned up once using SPRIselect reagent (Beckman Coulter B23319) using the manufacturers protocol. DNA was eluted in 18 uL and a first PCR using a i5 adapter forward primer and the ROS1 reverse primer pool was performed. Each PCR contained 10 uL DNA, 25 uL Platinum SuperFi 2x Master Mix (Invitrogen), 2.5ul of the i5 adapter forward primer and 2.5 uL of the ROS1 reverse primer pool. Cycling was followed using manufactures instructions. The PCR product was cleaned up once using SPRIselect reagent (Beckman Coulter B23319) using the manufacturers protocol. DNA was eluted in 18 uL and a second PCR using Indexed illumina primers was performed. Each PCR contained 15 uL DNA, 17.5 uL Platinum SuperFi 2x Master Mix (Invitrogen) and 2.4 uL Indexed illumina primers. PCR Cycling was followed using manufactures instructions. The PCR product was cleaned up once using SPRIselect reagent (Beckman Coulter B23319) using the manufacturers protocol. Indexed samples from different replicates were pooled into a tube containing 10uL lOmM Tris-HCl pH 8. Samples were selected for 195-350 bp using a 2% Agarose Dye Free cassette and marker L on the Pippin Prep (Sage Science), following the manufacturer's instructions. Size selected DNA was quantified by qPCR using a KAPA Library quantification kit (KAPABIOSYSTEMS), following the manufacturer's instructions. Quantified libraries were sequenced on the NextSeq500 Illumina platform and data analysis was performed.

Example 4: matching sequences to a reference database

Once sequence reads have been demultiplexed, adaptors have been trimmed and reads have been merged, they are compared against a database of all primers used in the fusion assay. Any sequencing reads containing the sequence of a primer designed to a 5' partner at the start and that of a 3' partner at the end are identified as potential fusion reads and carried forward for further analysis. The table of primers also contains a list of the expected sequences downstream from each primer (based on the primer bind site in the targeted region as opposed to another part of the genome) and the number of bases that need to match following the end of the primer in order to attain either low or high confidence that the sequence being read belongs to the potential fusion partner. A fusion may be called when the sequence from at least one side is identified with high confidence as belonging to a possible fusion partner (e.g. ELM4) and the other side is identified as belonging with at least low confidence to a fusion partner (e.g. ALK). A fusion might be only called if this is either detected in duplicate reactions or if there are 2 or more reads where both sides are high confidence.

Example 5: Determine Changes of ALK Fusion over time

Six plasma samples from a NSCLC patient were taken during a course of treatment and Fusion analysis (as described) performed. The total sequence reads for an ALK fusion and 16 normalisation regions (NRs) were determined. This information was then used to determine the change of the ALK fusion over the course of the treatment. To generate a ratio of Fusion to normalisation regions, the following equation was used

Total number of reads for Fusion / Median reads of NRs = Fusion ratio

The Fusion ratio was then used to determine the changes of Fusions over the different points. To look at the change in fusions over time the following equation was used:

$$\text{Fusion Ratio for First Time point} / \text{Fusion Ratio for Time Point X} = \text{Fold Change}$$

In this example the Fusion decreases at time point 3 in response to therapy and then increases at time point 5 and 6 indicating resistance to current therapy. These results are shown in Fig. 10.

Example 6: Determine Changes of ALK Fusion over three time points

Three plasma samples from a NSCLC patient were taken during a course of treatment and Fusion analysis (as described) performed. The total sequence reads for an ALK fusion and 16 normalisation regions (NRs) were determined. The method described in example 5 was followed. In this example the Fusion increases over time suggesting a failure to respond to treatment. These results are shown in Fig. 10.

It will also be recognized by those skilled in the art that, while the invention has been described above in terms of preferred embodiments, it is not limited thereto. Various features and aspects of the above described invention may be used individually or jointly. Further, although the invention has been described in the context of its implementation in a particular environment, and for particular applications (e.g. cfDNA analysis) those skilled in the art will recognize that its usefulness is not limited thereto and that the present invention can be beneficially utilized in any number of environments and implementations where it is desirable to examine other samples.

**Claims**

1.  A method for quantifying DNA gene fusion molecules in a sample, comprising:

    (a) combining a test sample comprising cell-free DNA (cfDNA) obtained from the bloodstream of a human subject with a set of primers and a polymerase to produce a reaction mix, wherein the set of primers comprises:

        i. at least 20 fusion-specific forward primers, wherein the fusion-specific forward primers tile across the same strand of a first region in a reference human genome,
        ii. at least 20 fusion-specific reverse primers, wherein the fusion-specific reverse primers tile across the same strand in a second region of the reference human genome;
        wherein the first and second regions are on different chromosomes or are on the same chromosome but spaced apart by at least 10 kb; and
        iii. a reference primer pair, wherein the reference primer pair amplifies a different region of the reference human genome, wherein the region amplified by the reference primer pair is in the range of 40 bp to 160 bp;

    (b) thermocycling the reaction mix to produce PCR products that comprise:

        i. a reference amplicon that is produced by the reference primer pair, and
        ii. one or more fusion amplicons that are produced using the fusion-specific primers from fusion molecules in the cfDNA, wherein the fusion molecules correspond to a genomic rearrangement that fuses the first region with the second region in at least some cells of the subject; and

(c) sequencing the PCR products of (b) or amplification products thereof to produce sequence reads; and
(d) quantifying the relative abundance of the fusion molecules in the test sample by comparing the number of sequence reads corresponding to fusion molecules with of the number of sequence reads corresponding to the reference region, to produce a ratio.

**2.** The method of claim 1, wherein the method comprises:

separately analyzing a first test sample and a second test sample using the method of claim 1, to obtain a first ratio indicating the abundance of the fusion molecules in the first sample and a second ratio indicating the abundance of the fusion molecules in the second test sample, wherein the first and second test samples are obtained from the same subject at different time points; and
comparing the first and second ratios to determine if the abundance of the fusion molecules has changed over time,
optionally wherein the subject has cancer, and the subject has received a treatment for cancer between the first and second time points.

**3.** The method of any prior claim, wherein the method comprises separately analysing test samples obtained from the same subject on at least 3 different time points using the method of claim 1 to obtain a time-course of ratios indicating the abundance of the fusion molecules in the test samples over time,
optionally further comprising comparing the time course of ratios to the time course of an allele frequency of another mutation in the sample, where the mutation is a single nucleotide variation or in-del.

**4.** The method of any prior claim, wherein fusion-specific forward primers hybridize to ALK, RET, NTRK1, ROS1, BRAF, EGFR, NRG1 or MET and the fusion-specific reverse primers hybridize to a different gene.

**5.** The method of any prior claim, wherein:

the set of primers of (a) comprises a plurality of reference primer pairs, wherein each reference primer pair amplifies a different sequence of the reference human genome, wherein the regions amplified by the reference primer pairs are of different lengths and in the range of 40 bp to 160 bp;
the PCR products of (b) comprise a plurality of reference amplicons that are produced by the reference primer pairs and said one or more fusion amplicons; and
step (d) comprises quantifying the relative abundance of the fusion molecules in the test sample by comparing the number of sequence reads corresponding to fusion molecules with of the number of sequence reads corresponding to at least some of the reference regions, to produce a ratio,
optionally wherein the regions amplified by the reference primer pairs differ in length from one another by at least 5 nt.

**6.** The method of claim 5, wherein the set of primers comprises at least 10 reference primer pairs, or wherein the set of primers comprises 10 to 30 reference primer pairs.

**7.** The method of any of claims 4 to 6, wherein the relative abundance of the fusion molecules is quantified by comparing the number of sequence reads corresponding to fusion molecules with the median number of sequence reads corresponding to at least some of the reference regions.

**8.** The method of any of claims 4 to 7, wherein the relative abundance of the fusion molecules is quantified by: i. eliminating sequence reads corresponding to one or more of the reference sequences and ii. comparing the number of sequence reads corresponding to fusion molecules with the median number of remaining sequence reads that correspond to the reference regions.

**9.** The method of any of claims 4 to 8, wherein sequence reads corresponding to outlier reference regions are eliminated.

**10.** The method of any of claims 4 to 9, wherein the method comprises eliminating sequence reads corresponding to reference regions that are not closely matched with the one or more fusion amplicons in GC content and/or length.

**11.** The method of any prior claim, wherein the average interval between adj acent binding sites for the forward primers in the first region is no more than 100 bases.

**12.** The method of any prior claim, wherein the average interval between adjacent binding sites for the reverse primers in the second region is no more than 100 bases.

**13.** The method of any prior claim, wherein the method is done using replicate samples,

optionally further comprising calculating the variability between the ratios for the replicate samples at each time point, and using the variability at each timepoint to determine the confidence that a difference of ratios between different time points reflects change in abundance of the fusion molecules over time,
optionally further comprising calculating a standard error for the replicate samples at each time point, and using the standard errors to estimate the significance of a difference in ratios across different time points for the same patient.

**14.** The method of any of claims 2 to 13, further comprising providing a report indicating the relative abundance of the fusion molecules at different timepoints,
optionally wherein the relative abundance of the fusion molecules at different timepoints is shown as a graph.

**15.** The method of any prior claim, further comprising determining whether there are multiple fusion amplicons corresponding to the genomic rearrangement, and if identified, the presence of multiple fusion amplicons corresponding to the genomic rearrangement increases the confidence that at least some of the cells of the subject comprise the genomic rearrangement,
optionally wherein step (d) is done by comparing the number of sequence reads corresponding to one or more of the reference sequences to i. the number of sequence reads corresponding to each of the multiple fusion amplicons to produce multiple ratios, or ii. all of the multiple fusion amplicons to produce a single ratio.

**Patentansprüche**

**1.** Verfahren zum Quantifizieren von DNA-Genfusionsmolekülen in einer Probe, umfassend:

(a) Kombinieren einer Testprobe, die dem Blutkreislauf eines Menschen entnommene zellfreie DNA (cfDNA) umfasst, mit einem Satz Primer und einer Polymerase unter Erhalt eines Reaktionsansatzes, wobei der Satz Primer Folgendes umfasst:

i. wenigstens 20 fusionsspezifische Vorwärtsprimer, wobei sich die fusionsspezifischen Vorwärtsprimer über den gleichen Strang einer ersten Region in einem menschlichen Referenzgenom aneinanderreihen,
ii. wenigstens 20 fusionsspezifische Rückwärtsprimer, wobei sich die fusionsspezifischen Rückwärtsprimer über den gleichen Strang in einer zweiten Region des menschlichen Referenzgenoms aneinanderreihen, wobei sich die erste und die zweite Region auf unterschiedlichen Chromosomen oder auf dem gleichen Chromosom, jedoch in einem Abstand von wenigstens 10 kb voneinander befinden; und
iii. ein Referenz-Primerpaar, wobei mit dem Referenz-Primerpaar eine andere Region des menschlichen Referenzgenoms amplifiziert wird, wobei die mit dem Referenz-Primerpaar amplifizierte Region im Bereich von 40 bp bis 160 bp liegt;

(b) Thermocycling des Reaktionsansatzes unter Erhalt von PCR-Produkten, die Folgendes umfassen:

i. ein Referenz-Amplifikat, das mit dem Referenz-Primerpaar erhalten wird, und
ii. ein oder mehrere Fusion-Amplifikate, die unter Verwendung der fusionsspezifischen Primer aus Fusionsmolekülen in der cfDNA erhalten werden, wobei die Fusionsmoleküle einer genomischen Umstellung entsprechen, bei der die erste Region mit der zweiten Region in wenigstens einigen Zellen des Individuums fusioniert wird; und

(c) Sequenzieren der PCR-Produkte unter (b) oder von Amplifikationsprodukten davon unter Erhalt von Sequenz-Reads; und
(d) Quantifizieren der relativen Häufigkeit der Fusionsmoleküle in der Testprobe, indem die Zahl von Sequenz-Reads, die Fusionsmolekülen entsprechen, mit einer der Reihe von Sequenz-Reads, die der Referenzregion entsprechen verglichen wird, so dass ein Verhältnis erhalten wird.

**2.** Verfahren nach Anspruch 1, wobei das Verfahren Folgendes umfasst:

getrenntes Analysieren einer ersten Testprobe und einer zweiten Testprobe unter Verwendung des Verfahrens nach Anspruch 1, so dass sein erstes Verhältnis, das die Häufigkeit der Fusionsmoleküle in der ersten Probe anzeigt, und ein zweites Verhältnis, das die Häufigkeit der Fusionsmoleküle in der zweiten Testprobe anzeigt, gewonnen werden, wobei die erste und die zweite Testprobe dem gleichen Individuum zu unterschiedlichen Zeitpunkten entnommen werden; und

Vergleichen des ersten und des zweiten Verhältnisses zur Bestimmung, ob sich die Häufigkeit der Fusionsmoleküle mit der Zeit geändert hat,

gegebenenfalls wobei bei dem Individuum eine Krebserkrankung vorliegt und das Individuum eine Krebsbehandlung zwischen dem ersten und dem zweiten Zeitpunkt bekommen hat.

3. Verfahren nach einem vorherigen Anspruch, wobei das Verfahren getrenntes Analysieren von Testproben, die dem gleichen Individuum zu wenigstens 3 unterschiedlichen Zeitpunkten entnommen werden, unter Verwendung des Verfahrens nach Anspruch 1 umfasst, so dass ein Zeitverlauf von Verhältnissen erhalten wird, der die Häufigkeit der Fusionsmoleküle in den Testproben im Laufe der Zeit anzeigt,

gegebenenfalls ferner umfassend Vergleichen des Zeitverlaufs von Verhältnissen mit dem Zeitverlauf einer Allelhäufigkeit einer anderen Mutation in der Probe, wobei es sich bei der Mutation um eine Einzelnukleotidvariation oder In-del handelt.

4. Verfahren nach einem vorherigen Anspruch, wobei fusionsspezifische Vorwärtsprimer an ALK, RET, NTRK1, ROS1, BRAF, EGFR, NRG1 oder MET hybridisieren und die fusionsspezifischen Rückwärtsprimer an ein anderes Gen hybridisieren.

5. Verfahren nach einem vorherigen Anspruch, wobei:

der Satz Primer unter (a) mehrere Referenz-Primerpaare umfasst, wobei mit jedem Referenz-Primerpaar eine unterschiedliche Sequenz des menschlichen Referenzgenoms amplifiziert wird, wobei die mit den Referenz-Primerpaaren amplifizierten Regionen unterschiedliche Längen aufweisen und im Bereich von 40 bp bis 160 bp liegen;

die PCR-Produkte unter (b) mehrere Referenz-Amplifikate, die mit den Referenz-Primerpaaren erhalten werden, sowie die ein oder mehreren Fusion-Amplifikate umfassen; und

Schritt (d) Quantifizieren der relativen Häufigkeit der Fusionsmoleküle in der Testprobe umfasst, indem die Zahl von Sequenz-Reads, die Fusionsmolekülen entsprechen, mit einer der Reihe von Sequenz-Reads, die wenigstens einigen der Referenzregionen entsprechen, verglichen wird, so dass ein Verhältnis erhalten wird,

gegebenenfalls wobei sich die mit den Referenz-Primerpaaren amplifizierten Regionen in ihrer Länge voneinander um wenigstens 5 nt unterscheiden.

6. Verfahren nach Anspruch 5, wobei der Satz Primer wenigstens 10 Referenz-Primerpaare umfasst oder wobei der Satz Primer 10 bis 30 Referenz-Primerpaare umfasst.

7. Verfahren nach einem der Ansprüche 4 bis 6, wobei die relative Häufigkeit der Fusionsmoleküle quantifiziert wird, indem die Zahl von Sequenz-Reads, die Fusionsmolekülen entsprechen, mit der mittleren Zahl von Sequenz-Reads, die wenigstens einigen der Referenzregionen entsprechen, verglichen wird.

8. Verfahren nach einem der Ansprüche 4 bis 7, wobei die relative Häufigkeit der Fusionsmoleküle quantifiziert wird, indem: i. Sequenz-Reads, die einer oder mehreren der Referenzsequenzen entsprechen, eliminiert werden und ii. die Zahl von Sequenz-Reads, die Fusionsmolekülen entsprechen, mit der mittleren Zahl verbliebener Sequenz-Reads, die den Referenzregionen entsprechen, verglichen wird.

9. Verfahren nach einem der Ansprüche 4 bis 8, wobei Sequenz-Reads, die Ausreißer-Referenzregionen entsprechen, eliminiert werden.

10. Verfahren nach einem der Ansprüche 4 bis 9, wobei das Verfahren Eliminieren von Sequenz-Reads umfasst, die Referenzregionen entsprechen, die mit den ein oder mehreren Fusionsamplifikaten hinsichtlich GC-Gehalt und/oder Länge nicht gut übereinstimmen.

11. Verfahren nach einem vorherigen Anspruch, wobei der mittlere Abstand zwischen angrenzenden Bindungsstellen für die Vorwärtsprimer in der ersten Region nicht mehr als 100 Basen beträgt.

**12.** Verfahren nach einem vorherigen Anspruch, wobei der mittlere Abstand zwischen angrenzenden Bindungsstellen für die Rückwärtsprimer in der ersten Region nicht mehr als 100 Basen beträgt.

**13.** Verfahren nach einem vorherigen Anspruch, wobei das Verfahren unter Verwendung von Parallelproben erfolgt, gegebenenfalls ferner umfassend Berechnen der Variabilität zwischen den Verhältnissen für die Parallelproben an jedem Zeitpunkt und Verwenden der Variabilität an jedem Zeitpunkt zur Bestimmung der Konfidenz, dass ein Unterschied von Verhältnissen zwischen unterschiedlichen Zeitpunkten eine Änderung der Häufigkeit der Fusionsmoleküle mit der Zeit widerspiegelt, gegebenenfalls ferner umfassend Berechnen eines Standardfehlers für die Parallelproben an jedem Zeitpunkt und Verwenden der Standardfehler zur Abschätzung der Signifikanz eines Unterschieds der Verhältnisse über unterschiedliche Zeitpunkte für den gleichen Patienten.

**14.** Verfahren nach einem der Ansprüche 2 bis 13, ferner umfassend Bereitstellen eines Berichts, der die relative Häufigkeit der Fusionsmoleküle zu unterschiedlichen Zeitpunkten angibt, gegebenenfalls wobei die relative Häufigkeit der Fusionsmoleküle zu unterschiedlichen Zeitpunkten als Graph dargestellt ist.

**15.** Verfahren nach einem vorherigen Anspruch, ferner umfassend Bestimmen, ob es mehrere Fusionsamplifikate gibt, die der genomischen Umstellung entsprechen, und, falls identifiziert, das Vorliegen mehrerer Fusionsamplifikate, die der genomischen Umstellung entsprechen, die Konfidenz erhöht, dass wenigstens einige der Zellen des Individuums die genomische Umstellung umfassen, gegebenenfalls wobei Schritt (d) erfolgt, indem die Zahl von Sequenz-Reads, die einer oder mehreren der Referenzsequenzen entsprechen, mit i. der Zahl von Sequenz-Reads, die jeweils den mehreren Fusionsamplifikaten entspricht, verglichen wird, so dass mehrere Verhältnisse erhalten werden, oder ii. allen der mehreren Fusionsamplifikate, verglichen wird, so dass ein einziges Verhältnis erhalten wird.

**Revendications**

**1.** Procédé pour la quantification de molécules de fusion de gène d'ADN dans un échantillon, comprenant :

(a) la combinaison d'un échantillon de test comprenant de l'ADN acellulaire (cfADN) obtenu à partir du flux sanguin d'un sujet humain avec un ensemble d'amorces et une polymérase pour produire un mélange de réaction, l'ensemble d'amorces comprenant :

i. au moins 20 amorces sens spécifiques à une fusion, les amorces sens spécifiques à une fusion étant disposées sur le même brin d'une première région dans un génome humain de référence,
ii. au moins 20 amorces antisens spécifiques à une fusion, les amorces antisens spécifiques à une fusion étant disposées sur le même brin dans une deuxième région du génome humain de référence ;
les première et deuxième régions étant sur des chromosomes différents ou étant sur le même chromosome mais distancées l'une de l'autre d'au moins 10 kb ; et
iii. une paire d'amorces de référence, la paire d'amorces de référence amplifiant une région différente du génome humain de référence, la région amplifiée par la paire d'amorces de référence étant dans la plage de 40 bp à 160 bp ;

(b) le thermocyclage du mélange de réaction pour produire des produits de PCR qui comprennent :

i. un amplicon de référence qui est produit par la paire d'amorces de référence ; et
ii. un ou plusieurs amplicons de fusion qui sont produits en utilisant les amorces spécifiques à une fusion provenant de molécules de fusion dans le cfADN, les molécules de fusion correspondant à un réarrangement génomique qui fusionne la première région avec la deuxième région dans au moins une partie des cellules du sujet ; et

(c) le séquençage des produits de PCR de (b) ou de produits d'amplification correspondants pour produire des lectures de séquences ; et
(d) la quantification de l'abondance relative des molécules de fusion dans l'échantillon de test en comparant le nombre de lectures de séquences correspondant à des molécules de fusion avec le nombre de lectures de séquences correspondant à la région de référence, pour produire un rapport.

**2.** Procédé selon la revendication 1, le procédé comprenant :

l'analyse de manière distincte d'un premier échantillon de test et d'un deuxième échantillon de test en utilisant le procédé selon la revendication 1, pour obtenir un premier rapport indiquant l'abondance des molécules de fusion dans le premier échantillon et un deuxième rapport indiquant l'abondance des molécules de fusion dans le deuxième échantillon de test, les premier et deuxième échantillons de test étant obtenus du même sujet à différents points temporels ; et
la comparaison des premier et deuxième rapports pour déterminer si l'abondance des molécules de fusion a changé dans le temps,
éventuellement, le sujet étant atteint d'un cancer, et le sujet ayant reçu un traitement pour le cancer entre les premier et deuxième points temporels.

**3.** Procédé selon une quelconque revendication précédente, le procédé comprenant l'analyse de manière distincte d'échantillons de test obtenus du même sujet en au moins 3 points temporels différents en utilisant le procédé selon la revendication 1 pour obtenir une évolution temporelle de rapports indiquant l'abondance des molécules de fusion dans les échantillons de test dans le temps,
éventuellement comprenant en outre la comparaison de l'évolution temporelle de rapports à l'évolution temporelle d'une fréquence allélique d'une autre mutation dans l'échantillon, où la mutation est une variation nucléotidique unique ou une in-del.

**4.** Procédé selon une quelconque revendication précédente, les amorces sens spécifiques à une fusion s'hybridant à ALK, RET, NTRK1, ROS1, BRAF, EGFR, NRG1 ou MET et les amorces antisens spécifiques à une fusion s'hybridant à un gène différent.

**5.** Procédé selon une quelconque revendication précédente,

l'ensemble d'amorces de (a) comprenant une pluralité de paires d'amorces de référence, chaque paire d'amorces de référence amplifiant une séquence différente du génome humain de référence, les régions amplifiées par les pairs d'amorces de référence étant de différentes longueurs et dans la plage de 40 bp à 160 bp ;
les produits de PCR de (b) comprenant une pluralité d'amplicons de référence qui sont produits par les paires d'amorces de référence et ledit ou lesdits amplicons de fusion ; et

l'étape (d) comprenant la quantification de l'abondance relative des molécules de fusion dans l'échantillon de test en comparant le nombre de lectures de séquences correspondant à des molécules de fusion au nombre de lectures de séquences correspondant à au moins une partie des régions de référence, pour produire un rapport,
éventuellement, les régions amplifiées par les pairs d'amorces de référence différant en longueur l'une par rapport à l'autre d'au moins 5 nt.

**6.** Procédé selon la revendication 5, l'ensemble d'amorces comprenant au moins 10 paires d'amorces de référence, ou l'ensemble d'amorces comprenant 10 à 30 paires d'amorces de référence.

**7.** Procédé selon l'une quelconque des revendications 4 à 6, l'abondance relative des molécules de fusion étant quantifiée en comparant le nombre de lectures de séquences correspondant à des molécules de fusion avec le nombre moyen de lectures de séquences correspondant à au moins une partie des régions de référence.

**8.** Procédé selon l'une quelconque des revendications 4 à 7, l'abondance relative des molécules de fusion étant quantifiée : i. en éliminant des lectures de séquences correspondant à l'une ou plusieurs parmi les séquences de référence et ii. en comparant le nombre de lectures de séquences correspondant à des molécules de fusion au nombre moyen de lectures de séquences restantes qui correspondent aux régions de référence.

**9.** Procédé selon l'une quelconque des revendications 4 à 8, des lectures de séquences correspondant à des régions de référence aberrantes étant éliminées.

**10.** Procédé selon l'une quelconque des revendications 4 à 9, le procédé comprenant l'élimination de lectures de séquences correspondant à des régions de référence qui ne correspondent pas étroitement à l'amplicon ou aux amplicons de fusion en teneur en GC et/ou en longueur.

**11.** Procédé selon une quelconque revendication précédente, l'intervalle moyen entre des sites de liaison adjacents pour les amorces sens dans la première région n'étant pas de plus de 100 bases.

**12.** Procédé selon une quelconque revendication précédente, l'intervalle moyen entre des sites de liaison adjacents pour les amorces antisens dans la deuxième région n'étant pas de plus de 100 bases.

**13.** Procédé selon une quelconque revendication précédente, le procédé étant réalisé en utilisant des échantillons répétés,

éventuellement comprenant en outre le calcul de la variabilité entre les rapports pour les échantillons répétés à chaque point temporel, et l'utilisation de la variabilité à chaque point temporel pour déterminer la confiance dans le fait qu'une différence de rapports entre différents points temporels reflète un changement d'abondance des molécules de fusion dans le temps,
éventuellement comprenant en outre le calcul d'un écart type pour les échantillons répétés à chaque point temporel, et l'utilisation des écarts types pour estimer l'importance d'une différence dans des rapports à des points temporels différents pour le même patient.

**14.** Procédé selon l'une quelconque des revendications 2 à 13, comprenant en outre la fourniture d'un rapport indiquant l'abondance relative des molécules de fusion à différents points temporels,
éventuellement, l'abondance relative des molécules de fusion à différents points temporels étant représentée sur un graphique.

**15.** Procédé selon une quelconque revendication précédente, comprenant en outre la détermination du fait qu'il y a, ou non, de multiples amplicons de fusion correspondant au réarrangement génomique, et si elle est identifiée, la présence de multiples amplicons de fusion correspondant au réarrangement génomique augmentant la confiance dans le fait qu'au moins une partie des cellules du sujet comprennent le réarrangement génomique,
éventuellement, l'étape (d) étant réalisée en comparant le nombre de lectures de séquences correspondant à l'une ou plusieurs parmi les séquences de référence i. au nombre de lectures de séquences correspondant à chacun des multiples amplicons de fusion pour produire de multiples rapports, ou ii. à tous les multiples amplicons de fusion pour produire un unique rapport.

Control Wt DNA ⟍ CellLine Fusion mix ⟋ EML4-ALK Positive Cell line

EML4-ALK Fusion at 1, 0.5, 0.25. 0.125, 0.0625%

↓

Fusion Enrichment

↓

Next Generation Sequencing

↓

Analysis

↓

Fusion Detection Report

**Fig. 1**

EP 3 894 600 B1

A

## Expected EML4-ALK Breakpoint

GAAGTTCCTATACTTTCTAGAGAATAGGAACTTC

## Primer Set 1: Read obtained:

TTAATTAAATGTTTTTCAAATCCATTCACCTGAATGTCTAAGCTTGGCAGGAAGTTCCTATACTTTCTAGAGAATAGGAACTTCGGAATAGGAACTTCATATGGTGCC

## Primer Set 2: Read obtained:

AGGAAGTTCCTATACTTTCTAGAGAATAGGAACTTCGGAATAGGAACTTCATATGGTGCCATCCCTCA

B

**Fig. 2**

EP 3 894 600 B1

Fig. 3

Control Wt DNA ———————→ 1% Fusion Event ←——————— gBlock ROS1-CD74

↓

Fragmentation to 150bp

↓

Fusion Enrichment

↓

Next Generation Sequencing

↓

Analysis

↓

Fusion Detection Report

**Fig. 4**

CD74-ROS1 gBlock Sequence

CGCCAAGGTCACAGCTGCTGCCAAGAGAGCCTTGGGCGTTTCCCACCTCATG
GACAATGCAGACTAGGATGTTTTTAGACCCAAAGACAGAGTGCTGTTTCTATC
CCGGTGCTGTCTCTAATTTGCTATGTAACTTTGGACAAGTCCCCTTCCCTCTAG
GATTCAGGGTCCTGAAGTAGAAGGTCAAAGGGCCACCCTGCCTGGGGCCTC
AGTTTCTGCATCAGATTCATAGAAGGCACCTTACATGCT[AT]TGTCATGTATATT
TTCTGTATTTATTATTTTTTGCAGAAAGAGCACTTCAAATAATTTACAGAACCA
GAATTTAAGGTGGAGATGACATTTAATGGATCCTGCAGTAGTGTTTGCACATG
GAAGTCCAAAAACCTGAAAGGAATATTTCAGTTCAGAGTAGTAGCTGCAAAT
AATCTAGGGTTTGGTGAATATAGTGGAATCAGTGAGAATATTATATTAGTTGGA
GGTATGTTACCATGTCTGTCTA

CD74

ROS1

[Insertion]

**Fig. 5**

EP 3 894 600 B1

A

| Forward Primer | Reverse Primer | |
|---|---|---|
| CD74_E6-I6_10 | ROS1_I32_E33_414 | |
| CD74_E6-I6_9 | ROS1_I32_E33_414 | |

**Read Sequence**

TTACATGCTATTGTCATGTATATTTTCTGTATTTATTATTTTTTGCAGAAAGAGCACTTCAAATAAT
TTACAGAACCAGAATTTAAGGTGGAAGATGACATTTA

CCCTGCCTGGGGCCTCAGTTTCTGCATCAGATTCATAGAAGGCACCTTACATGCTATTGTCATGT
ATATTTTCTGTATTTATTATTTTTTGCAGAAAGAGCACTTCAAAT

B

## CD74_E6-I6_10/ROS1_I32_E33_414 Read

CGCCAAGGTCACAGCTGCTGCCAAGAGAGCCTTGGGCGTTTCCCA
CCTCATGGACAATGCAGACTAGGATGTTTTTAGACCCAAAGACAG
AGTGCTGTTTCTATCCCGGTGCTGTCTCTAATTTGCTATGTAACTTTG
GACAAGTCCCCTTCCCTCTAGGATTCAGGGTCCTGAAGTAGAAGG
TCAAAGGGCCA**CCCTGCCTGGGGCCTCAGTTTCTGCATCAGATTCA
TAGAAGGCACCTTACATGCT[AT]**<u>TGTCATGTATATTTTCTGTATTTAT
TATTTTTTGCAGAAAGAGCACTTCAAATAATTTACAGAACCAGAAT
TTAAGGTGGAGATGACATTTA</u>ATGGATCCTGCAGTAGTGTTTGCAC
ATGGAAGTCCAAAAACCTGAAAGGAATATTTCAGTTCAGAGTAGT
AGCTGCAAATAATCTAGGGTTTGGTGAATATAGTGGAATCAGTGAG
AATATTATATTAGTTGGAGGTATGTTACCATGTCTGTCTA

## CD74_E6-I6_9/ROS1_I32_E33_414 Read

CGCCAAGGTCACAGCTGCTGCCAAGAGAGCCTTGGGCGTTTCCCA
CCTCATGGACAATGCAGACTAGGATGTTTTTAGACCCAAAGACAG
AGTGCTGTTTCTATCCCGGTGCTGTCTCTAATTTGCTATGTAACTTTG
GACAAGTCCCCTTCCCTCTAGGATTCAGGGTCCTGAAGTAGAAGG
TCAAAGGGCCACCCTGCCTGGGGCCTCAGTTTCTGCATCAGATTCA
TAGAAGGCACC**TTACATGCT[AT]**<u>TGTCATGTATATTTTCTGTATTTAT
TATTTTTTGCAGAAAGAGCACTTCAAATAATTTACAGAACCAGAAT
TTAAGGTGGAGATGACATTTA</u>ATGGATCCTGCAGTAGTGTTTGCAC
ATGGAAGTCCAAAAACCTGAAAGGAATATTTCAGTTCAGAGTAGT
AGCTGCAAATAATCTAGGGTTTGGTGAATATAGTGGAATCAGTGAG
AATATTATATTAGTTGGAGGTATGTTACCATGTCTGTCTA

CD74   <u>ROS1</u>   [Insertion]   **Sequence Read**

**Fig. 6**

a) Normal cell

75bp

Gene B

Gene A

b) Fusion positive cancer cell

c) Second PCR amplification

Fig. 7

EP 3 894 600 B1

**A**   Strong Anchor: Sequence uniquely maps to Genome

Adapter   ALK   EML4   Adapter

breakpoint

Strong Anchor: Sequence uniquely maps to Genome

**B**

Strong Anchor: Sequence uniquely maps to Genome

Adapter   ALK   EML4   Adapter

breakpoint

Weak Anchor: Sequence does not uniquely map to Genome

**Fig. 8**

Fig. 9

A) High confidence change

B) Low confidence change

Fusion level

Timepoint

EXAMPLE 5 DETERMINE CHANGES IN ALK FUSION OVER TIME

FOLD CHANGE (T=0)

COLLECTION DATE

Fig. 10

EXAMPLE 6 DETERMINE CHANGES IN ALK FUSION OVER 3 TIMEPOINTS

Fig. 11

**EP 3 894 600 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018005983 A
- WO 2008144841 A
- WO 2018229514 A
- GB 2018051688 W
- GB 1709675 A

**Non-patent literature cited in the description**

- **GAO et al.** *Cell Reports,* 2018, vol. 23, 227-238
- **HARRIS et al.** *Nature Scientific Reports,* 2016, vol. 6, 29831
- **KORNBERG ; BAKER.** DNA Replication. W.H. Freeman, 1992
- **LEHNINGER.** Biochemistry. Worth Publishers, 1975
- **STRACHAN ; READ.** Human Molecular Genetics. Wiley-Liss, 1999
- Oligonucleotides and Analogs: A Practical Approach. Oxford University Press, 1991
- Oligonucleotide Synthesis: A Practical Approach. IRL Press, 1984
- **AUSUBEL et al.** Current Protocols in Molecular Biology. John Wiley & Sons, Inc,
- **SAMBROOK et al.** Molecular Cloning: A Laboratory Manual. Cold Spring Harbor Laboratory Press
- **AUSUBEL et al.** Short Protocols in Molecular Biology. Wiley & Sons, 1995
- **SAMBROOK et al.** Molecular Cloning: A Laboratory Manual. Cold Spring Harbor, 2001
- **LO et al.** *Am J Hum Genet,* 1998, vol. 62, 768-75
- **MARGULIES et al.** *Nature,* 2005, vol. 437, 376-80
- **RONAGHI et al.** *Analytical Biochemistry,* 1996, vol. 242, 84-9
- **SHENDURE.** *Science,* 2005, vol. 309, 1728
- **IMELFORT et al.** *Brief Bioinform.,* 2009, vol. 10, 609-18
- **FOX et al.** *Methods Mol Biol.,* 2009, vol. 553, 79-108
- **APPLEBY et al.** *Methods Mol Biol.,* 2009, vol. 513, 19-39
- **ENGLISH.** *PLoS One.,* 2012, vol. 7, e47768
- **MOROZOVA.** *Genomics,* 2008, vol. 92, 255-64
- **SEKI ; MIZUKAMI ; KOHNO.** *Biomolecules,* 2015, vol. 5, 2464-2476

36